# EUROPEAN PATENT APPLICATION

(11) **EP 4 141 026 A1**
(43) Date of publication of application: **01.03.2023**
(21) Application number: 21193927.7
(22) Date of filing: 31.08.2021
(51) Int. Cl.: C07K 16/18, C07K 14/47, A61P 37/00

(54) **FUSION PROTEINS COMPRISING ANTI C3B SINGLE DOMAIN ANTIBODY FOR COMPLEMENT REGULATION**

(71) Applicant: Aarhus Universitet, 8000 Aarhus C (DK)
(72) Inventor: Stub Laursen, Nick, 8000 Aarhus C (DK); Pedersen, Henrik, 8000 Aarhus C (DK); Kjeldsen Jensen, Rasmus, 69126 Heidelberg (DE); Rom Andersen, Gregers, 8220 Brabrand (DK)
(74) Representative: Høiberg P/S

(57) **Abstract**

The present application relates to a fusion protein comprising: a single domain antibody that binds to C3b; a polypeptide comprising or consisting of the amino acid sequence according to SEQ ID NO: 10, or a variant thereof having at least 80% sequence identity to the amino acid sequence according to SEQ ID NO: 10; and a first linker. Furthermore is provided polypeptides, nucleic acids, vectors and methods of preparing these as well as methods of treatment and pharmaceutical compositions.

## Description

### Technical field

The present invention relates to fusion proteins comprising single domain antibodies and recombinant proteins comprising complement control protein (CCP) domains.

### Background

The complement system constitutes an important arm of the innate immune system. The complement system labels - or opsonizes - intruding pathogens as well as dying host cells for clearance. Excess activation of the complement system may lead to development of a number of diseases. The complement system hence emerges as an attractive target for inhibitors to treat or ameliorate these diseases.

The complement system comprises the three pathways: the classical pathway, the lectin pathway and the alternative pathway (AP). In the initiating steps of the complement system, pattern recognition molecules (PRM) of the classical and lectin pathways binds to the activator. This leads to activation of PRM-associated serine proteases that cleaves complement component C4. One of the cleavage products of C4, C4b covalently attaches to the activator and associates with C2. Upon proteolytic activation of C2, this complex form the C4b2a C3 convertase, that cleaves C3. One of the cleavage products, C3b covalently attaches to the activator and associates with factor B. The C3b-bound factor B may be activated by factor D, which liberates the Ba moiety from the C3b-bound Bb moiety and leads to the formation of the alternative pathway C3 convertase, C3bBb. The AP C3 convertase cleaves C3 and thereby amplifies the outcome of the two other pathways (Merle et al., 2015).

The AP may also initiate independently of the classical and lectin pathways, through spontaneous hydrolysis of an internal thioester of C3. The resulting C3(H2O) associates with factor B, which is subsequently cleaved by factor D. The resulting complex C3(H2O)Bb is a fluid phase C3 convertase, that cleaves C3 into C3a and C3b. The nascent C3b may react with nucleophiles on surfaces, whereby the fluid phase C3 convertase, in principle, may lead to opsonization of any surface.

To prevent excess complement activation, the system also comprises a number of regulators. One of these regulators, factor H, is a fluid phase regulator that interacts with host cells and inhibits the complement progression in two ways. Firstly, factor H is a cofactor for the serine protease factor I that degrades and irreversibly inactivates the activation product of the central complement C3b. Secondly, factor H dissociates the alternative pathway C3 convertase. Factor H comprises 20 CCP domains.

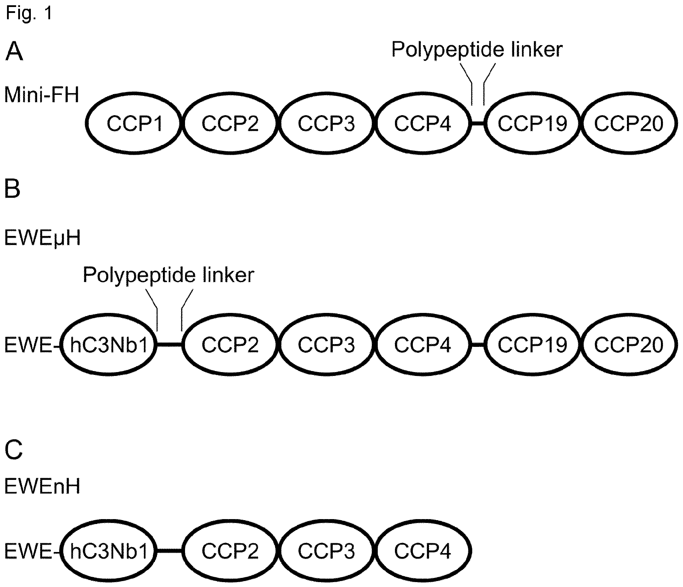

Different minimized versions of factor H have previously been described, including the mini-FH comprising CCP1-4 and CCP19-20 joined by a polypeptide linker (Fig 1) (WO 2013/142362 Al). CCP1-3 is believed to be the minimal unit with cofactor activity for C3b proteolysis by factor I (Hocking et al. 2008). The single domain antibody named hC3Nb1 capable of binding C3 and C3b is disclosed in Jensen et al. 2018. A C3b specific recombinant version of this single domain antibody named EWE-hC3Nb1 is described in WO 2019/238674 A1.

### Summary

Regulators of the complement system have long been sought for, since overactivation of the complement system induces tissue damage and sustains chronic inflammation. The inventors of the present disclosure made the surprising discovery that a recombinant linkage of EWE-hC3Nb1 to a novel minimized version of factor H comprising the CCP2-4 and CCP19-20 domains of factor H, results in an AP inhibitor that mediates cleavage of C3b by factor I. As shown in the examples, this novel fusion protein retains both its capabilities from the single domain antibody moiety and surprisingly also the recombinant factor H moiety, thus showing that the inventors have in fact discovered an AP inhibitor that mediates cleavage of C3b by factor I. Having seen the surprising effect of this fusion protein, the inventors of the present disclosure went on to develop an even further minimized version comprising only EWE-hC3Nb1 and CCP2-4. This smaller protein showed an even higher efficiency in sustaining C3b cleavage, a slightly lower binding affinity towards C3b and a high capability of being concentrated to a high concentration.

Thus, in a first aspect, the disclosure relates to a fusion protein comprising:
a) a single domain antibody that binds to C3b;
b) a polypeptide comprising or consisting of the amino acid sequence according to SEQ ID NO: 10, or a variant thereof having at least 80% sequence identity to the amino acid sequence according to SEQ ID NO: 10; and
c) a first linker.

Having realized that the factor H mediated cofactor activity for C3b proteolysis by factor I was retained in the novel minimized version comprising only CCP2-4 and the version comprising CCP2-4 and CCP19-20, the invention in two further aspects relates to:
A polypeptide having a length of less than 220 amino acids, wherein the polypeptide comprises the amino acid sequence according to SEQ ID NO: 10 or a variant thereof having at least 80% sequence identity to the amino acid sequence according to SEQ ID NO: 10; and
A polypeptide having a length of less than 350 amino acids, wherein the polypeptide comprises the amino acid sequence according to SEQ ID NO: 9 or a variant thereof having at least 80% sequence identity to the amino acid sequence according to SEQ ID NO: 9.

In further aspects, the disclosure also relates to a nucleic acid encoding the fusion protein or the polypeptides of the present disclosure.

In a further aspect, the disclosure relates to a vector comprising the nucleic acid of the disclosure.

In a further aspect, the disclosure relates to a method of preparing the vector of the disclosure, the method comprising:
a) introducing the vector of the disclosure into a cell;
b) culturing the cell; and
c) collecting and purifying the vector from the cell;
d) optionally, lysing the cell to release the vector from the cell.

In a further aspect, the disclosure relates to a host cell comprising the nucleic acid or the vector of the disclosure.

In a further aspect, the disclosure relates to a method of preparing the fusion protein or the polypeptide of the disclosure, the method comprising:
a) introducing the nucleic acid or the vector of the disclosure into a host cell;
b) culturing the cell; and
c) obtaining and optionally purifying the fusion protein or polypeptide from the supernatant;
d) optionally, lysing the cell prior to purifying the fusion protein or the polypeptide.

In a further aspect, the disclosure relates to a pharmaceutical composition comprising the fusion protein or the polypeptide of the disclosure, optionally comprising a pharmaceutically acceptable carrier.

In a further aspect, the disclosure relates to a method of treating a disorder associated with complement activation, the method comprising administering a therapeutically effective amount of the fusion protein, the polypeptide or the pharmaceutical composition of the disclosure to a subject in need thereof.

In a further aspect, the disclosure relates to a method of modulating the activity of the complement system, the method comprising: administering to an individual in need thereof a therapeutically effective amount of the fusion protein, the polypeptide or the pharmaceutical composition of the disclosure, thereby modulating the activity of the complement system in the individual in need thereof.

### Description of Drawings

**Figure 1** **Schematic representation of minimized factor H fragments. (A)** The previously described mini-FH comprising CCP domains 1-4 and 19-20 of factor H. (B) The EWEµH fragment (SEQ ID NO: 5) described in the present disclosure, comprising the single domain antibody, EWE-hC3Nb1 (SEQ ID NO: 1), as well as CCP domains 2-4 and 19-20 of factor H (SEQ ID NO: 9). (C) The EWEnH (SEQ ID NO: 7) described in the present disclosure. EWEnH comprises the single domain antibody, EWE-hC3Nb1 (SEQ ID NO: 1) and CCP domains 2-4 of factor H (SEQ ID NO: 10).

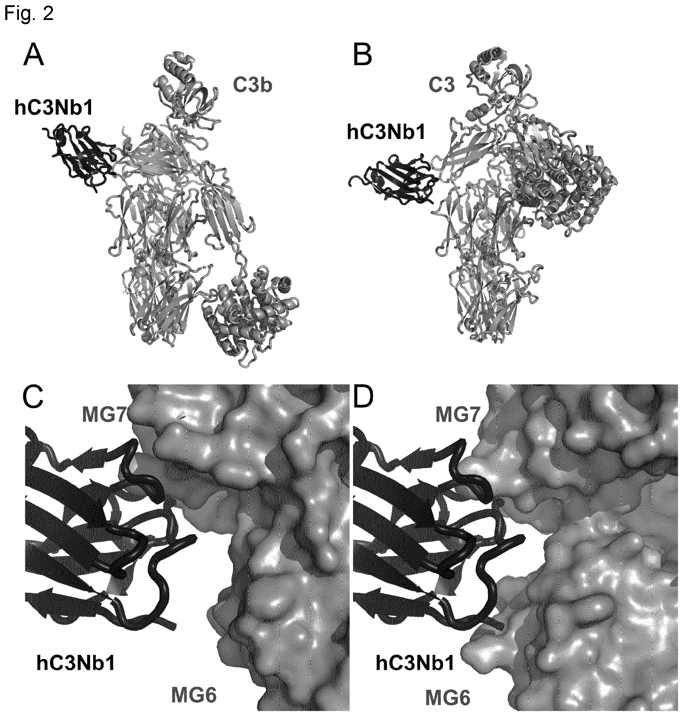

**Figure 2** **Structural comparison of hC3Nb1 in complex with C3b and native C3.** Structure of the parental hC3Nb1 (black) in complex with **(A)** C3b (grey) [PDB entry 6EHG] and **(B)** native C3 (grey) [PDB entry 6RU5]. The C3 molecules of panels A and B are aligned on the macroglobulin (MG)-ring. The binding interface between hC3Nb1 and **(C)** C3b [PDB entry 6EHG] and **(D)** native C3 [PDB entry 6RU5]. The structures in panels C and D are aligned on the hC3Nb1 molecule.
   When comparing (C) and (D) the figure shows that the MG6 domain is in close proximity to the hC3Nb1 single domain antibody in (D) whereas the MG6 domain is further away in (C). On the basis of this discovery the inventors hypothesized that an additional N-terminal motif might confer specificity towards C3b (C).

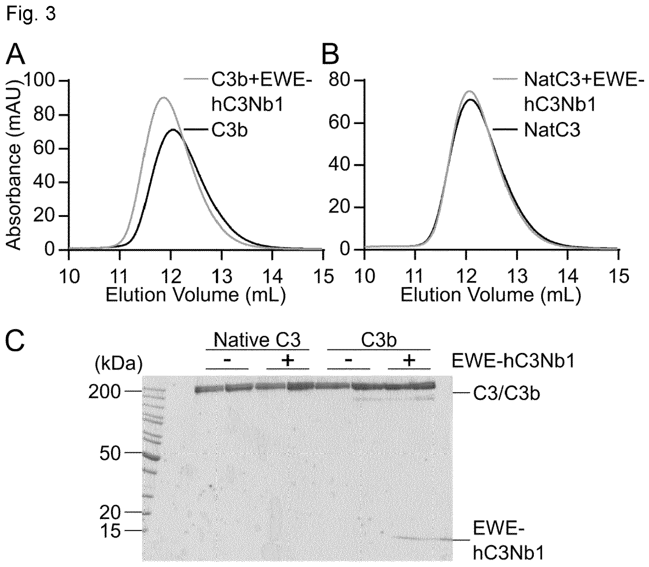

**Figure 3** **Validation of EWE-hC3Nb1. (A)** SEC-based analysis of the C3b:EWE-hC3Nb1 interaction. C3b in presence or absence of EWE-hC3Nb1 was subjected to SEC. **(B)** As in panel A, but analyzing the C3:EWE-hC3Nb1 interaction. **(C)** Nonreducing SDS-PAGE analysis of peak fractions from panels A-B. The data were previously presented in WO 2019/238674 A1.
   The figure shows that EWE-hC3Nb1 conferred a clear shift in elution volume, when mixed with C3b (A), but not when mixed with native C3 (B), showing that EWE-hC3Nb1 is specific against C3b. Subsequent SDS PAGE of peak fractions confirmed the formation of the EWE-hC3Nb1:C3b complex (C). On the basis of these data, the inventors concluded that the addition of the N-terminal motif had in fact created specificity against C3b.

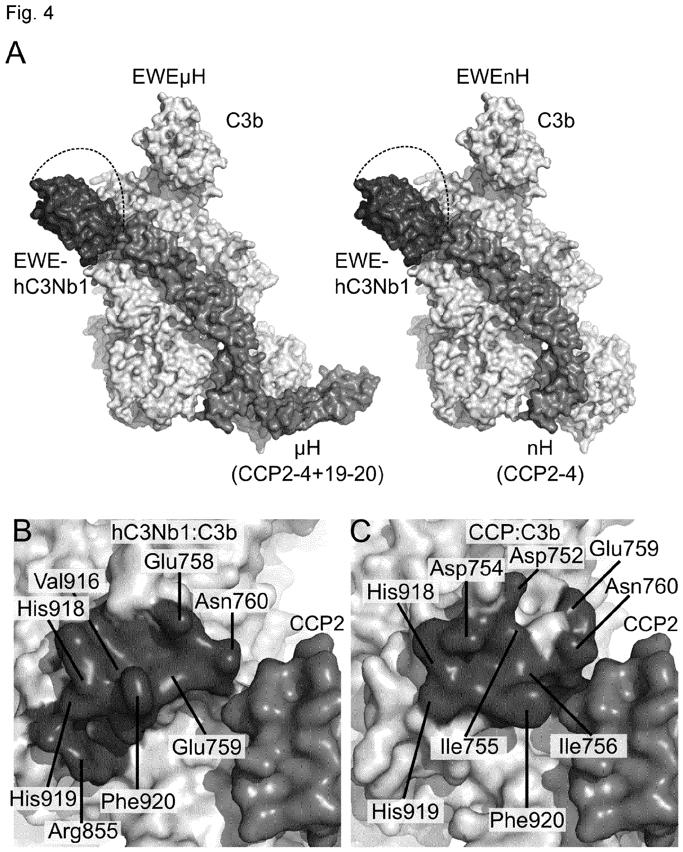

**Figure 4** **Design of EWEµH and EWEnH. (A)** *left:* The structure of hC3Nb1 [PDB entry 6EHG] (Jensen et al., 2018) was superimposed onto the structure of C3b:mini-FH [PDB entry 5O35] (Xue et al., 2017) without the CCP1 domain. The dashed line illustrates a flexible linker connecting the C-terminus of EWE-hC3Nb1 and the N-terminus of the CCP2 domain. *Right:* The structure of hC3Nb1 [PDB entry 6EHG] (Jensen et al., 2018) was superimposed onto the structure of C3b:mini-FH [PDB entry 5O35] (Xue et al., 2017) without the CCP1, and CCP19-20 domains. The dashed line illustrates a flexible linker connecting the C-terminus of EWE-hC3Nb1 and the N-terminus of the CCP2 domain. **(B)** Interaction site of hC3Nb1 on C3b indicated as footprint [PDB entry 6EHG]. **(C)** Interaction site of CCP1 of mini-FH on C3b indicated as footprint [PDB entry 5O35]. Amino acid residues within 4.5 Å of hC3Nb1 and CPP1 are indicated in panels B and C, respectively.
   The figure shows how fusion proteins of the disclosure bind to C3b (A) and that EWE-hC3Nb1 and the FH CCP1 contact C3b in the same region and to some extent interact with identical residues on C3b (B) and (C). On the basis of these data, the inventors concluded that EWE-hC3Nb1 and the FH CCP1 domain might compete for binding to C3b.

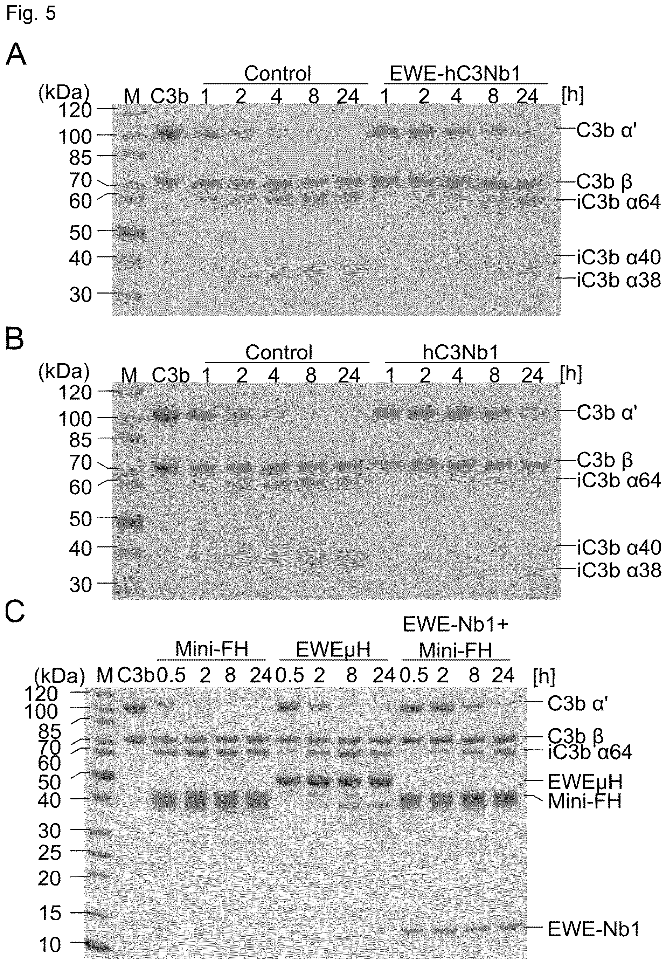

**Figure 5** **Initial validation of EWEµH. (A-B)** Factor H-mediated C3b cleavage by factor I. C3b was incubated with 0.2% factor H, 1% factor I and a 1.2-fold molar excess of either (A) EWE-hC3Nb1 or (B) hC3Nb1. The data in panel A were previously presented in WO 2019/238674 A1. The cleavage of C3b was monitored by SDS PAGE. (C) EWEµH cleavage assay. C3b was incubated in presence of factor I and a two-fold molar excess of mini-FH, EWEµH or mini-FH as well as EWE-hC3Nb1. The cleavage of C3b was monitored by SDS-PAGE analysis upon the indicated incubation at 37°C.
   The figure shows that EWE-hC3Nb1 (A) and hC3Nb1 (B) delays the cleavage of C3b by factor I. In addition, EWEµH (C) compared to mini-FH (CCP1-4, 19-20) as well as a mix of both EWE-hC3Nb1 and mini-FH (CCP1-4, 19-20) shows that EWEµH sustains the cleavage of C3b, however that the mix of EWE-hC3Nb1 and mini-FH does not. On the basis of these data, the inventors concluded that EWEµH mediates cleavage of C3b by factor I.

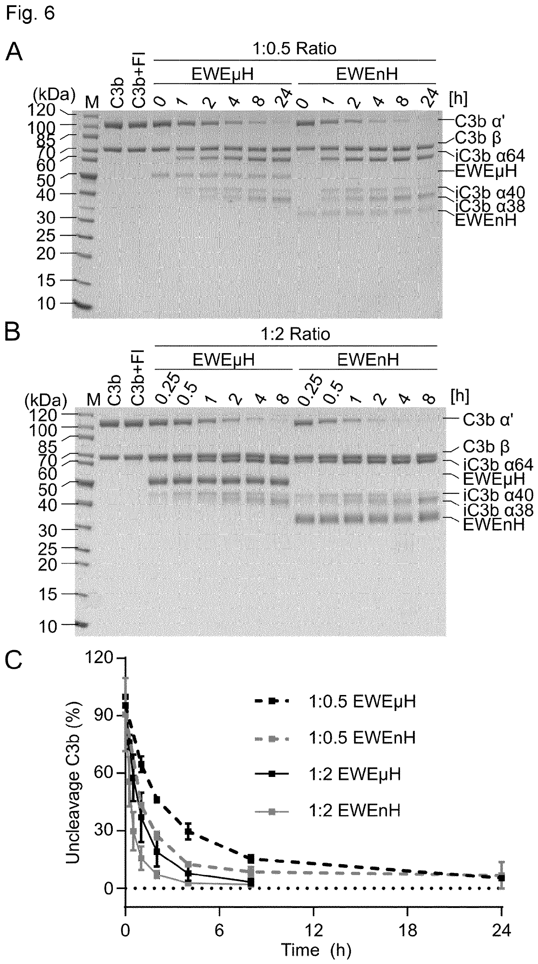

**Figure 6** **Comparison of EWEµH and EWEnH. (A-B)** Comparison of EWEµH and EWEnH. C3b was incubated with 0.5% factor I and a (A) 0.5-fold molar ratio or (B) 2-fold molar excess of EWEµH or EWEnH. The cleavage of C3b by factor I was monitored by SDS-PAGE analysis upon the indicated period of incubation at 37°C. (C) Quantification of cleavage. The intensity of the bands of the a'-chain of C3b in panel A-B were quantified using the ImageJ software. The intensity was normalized to the intensity of the a'-chain of C3b incubated with factor I for 8 h. Average and S.D. (error bars) are shown for n=3 experiments for the 1:2 ratio and n=2 experiments for the 1:0.5 ratio.
   The figure shows that EWEnH and EWEµH mediates cleavage of C3b by factor I in molar ratios of C3b:fusion protein of 1:0.5 (A) and 1:2 (B). The quantitive comparison in (C) indicates that EWEnH is more efficient in sustaining C3b cleavage at both tested molar ratios. These data conclude that both fusion proteins are capable of mediating cleavage of C3b, and that EWEnH might be more efficient.

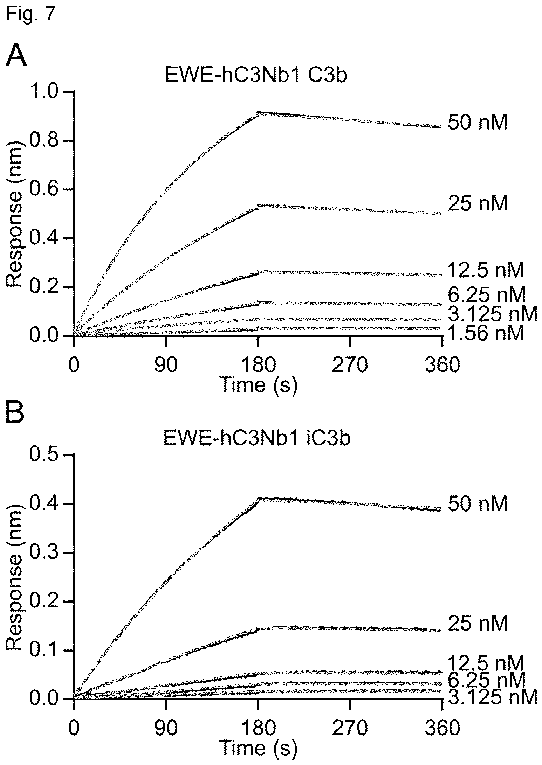

**Figure 7** **Bio-layer interferometry-based analysis of EWE-hC3Nb1. (A)** Anti-penta his biosensors were coated with EWE-hC3Nb1 and the sensors were transferred into a dilution series from 1.56-50 nM C3b. **(B)** Anti-penta his biosensors were coated with EWE-hC3Nb1 and transferred into a dilution series of 3.125-50 nM iC3b.
   The figure shows that EWE-hC3Nb1 exhibits similar affinity toward C3b (A) and iC3b (B).

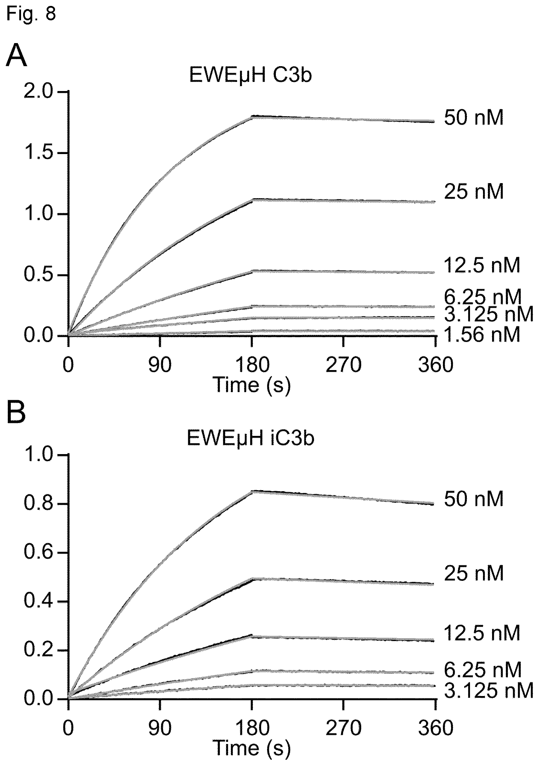

**Figure 8** **Bio-layer interferometry-based analysis of EWEpH.** (A) Anti-penta his biosensors were coated with EWEµH. The sensors were transferred into a dilution series from 1.5625-50 nM C3b. **(B)** Anti-penta his biosensors were coated with EWEµH and transferred into an iC3b dilution series ranging from 3.125-50 nM.
   The figure shows that EWEµH exhibits higher affinity towards C3b (A) than towards iC3b (B).

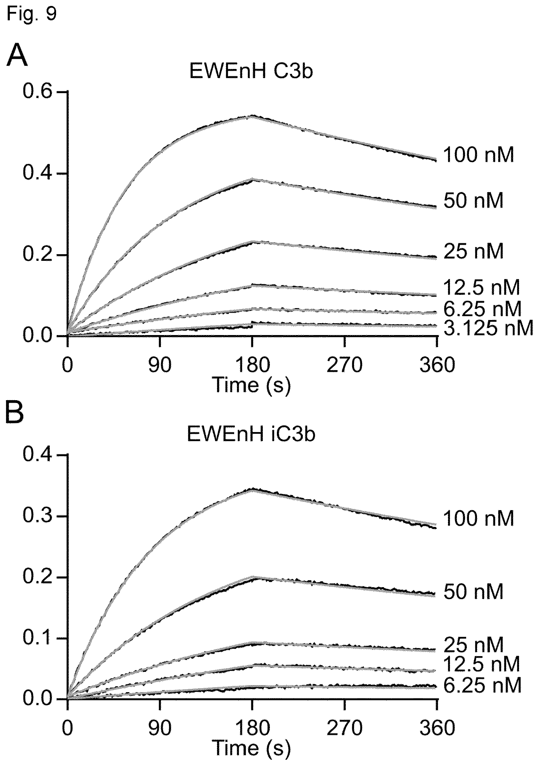

**Figure 9** **Bio-layer interferometry-based analysis of EWEnH. (A)** Anti-penta his biosensors were coated with EWEnH. The sensors were transferred into a dilution series from 3.125-100 nM C3b. **(B)** Anti-penta his biosensors were coated with EWEnH. The iC3b concentration tested ranged from 6.25-100 nM.
   The figure shows that EWEnH exhibits similar affinity toward C3b (A) and iC3b (B).

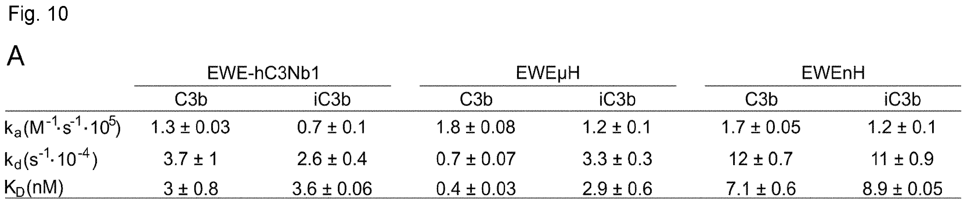

**Figure 10** **Summary of affinities. (A)** The table summarizes the mean of kₐ, k_{d} and K_{D} ± standard deviation from n=3 experiments for C3b kinetics and n=2 experiments for iC3b kinetics.
   The figure shows that the fusion EWEµH binds app. 10x stronger to C3b than the single domain antibody EWE-hC3Nb1, whereas it binds with similar affinity towards iC3b. The fusion protein EWEnH shows a slightly lower binding affinity towards C3b and iC3b than the single domain antibody EWE-hC3Nb1. Overall, these data conclude that strong binding affinity towards C3b and iC3b of the single domain antibody EWE-hC3Nb1 is retained when the single domain antibody is comprised in a fusion protein.

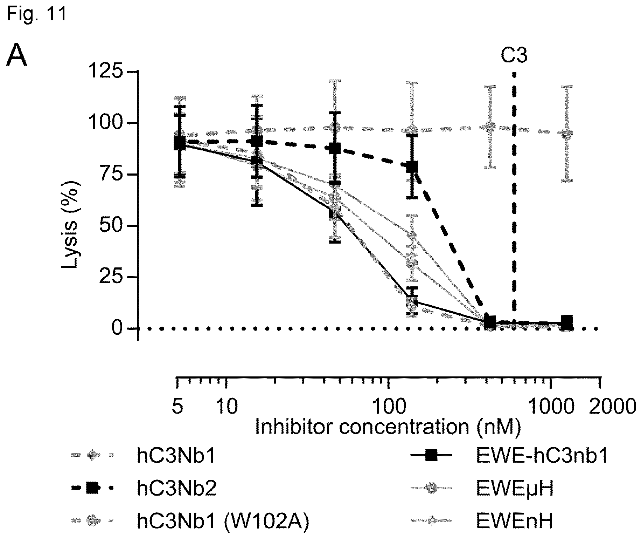

**Figure 11** **Functional comparison of EWEµH and EWEnH. (A)** Inhibition of the AP mediated hemolysis of rabbit erythrocytes. The effects of EWE-hC3Nb1, EWEµH, and EWEnH were assayed in 11% serum. The lysis was normalized to 100% in NHS without nanobodies, and 0% without NHS. The effects were compared to the parental AP inhibitor hC3Nb1 (Jensen et al., 2018), the broad inhibitor hC3Nb2 (Pedersen et al., 2020), and the inactive hC3Nb1 (W102A) mutant (Jensen et al., 2018). The dashed line indicate putative C3 concentrations assuming a C3 concentration of 5.4 µM in undiluted serum (Engstrom et al., 2007). Average and S.D. (error bars) are shown for n=3 experiments.
   The figure shows that EWEµH, EWEnH, EWE-hC3Nb1, and hC3Nb1 inhibit the AP mediated C3 fragment deposition. The data confirms that the functional properties of the single domain antibody EWE-hC3Nb1 is retained when the single domain antibody is comprised in a fusion protein.

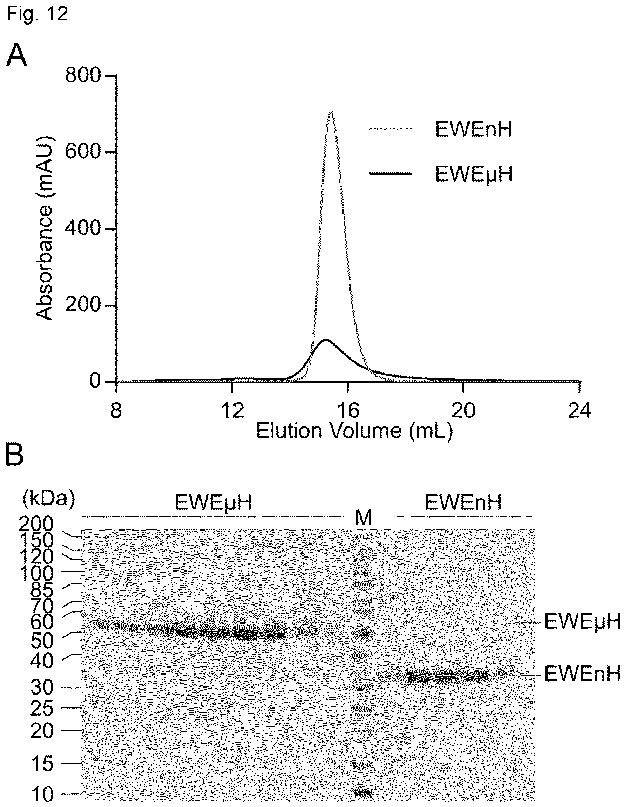

**Figure 12** **Concentration assay of EWEµH and EWEnH. (A)** EWEµH and EWEnH were concentrated and subjected to SEC. **(B)** SDS-PAGE analysis of peak fractions from panel C.
   The figure shows that the fusion protein EWEnH tolerates ultrafiltration to higher concentrations than EWEµH. On the basis of these data, the inventors conclude that the smaller protein EWEnH tolerates being ultra-filtrated to a surprisingly high concentration and thus might have a higher clinical and commercial value than EWEµH.

### Detailed description

The present invention relates to fusion proteins capable of targeting the complement C3b protein and facilitate its degradation. Generally, the fusion proteins of the disclosure comprise a single domain antibody, thus when single domain antibodies are described further below, they are understood as being comprised in the fusion protein. The previously described single domain antibody hC3Nb1 inhibits the AP by preventing the binding of FB to C3b. hC3Nb1 binds at the interface between MG6 and MG7 domains of C3b and C3 (Fig 2). In complex with C3, the N-terminus is in close proximity to the MG6 domain (Fig 2D). The inventors rationalized that an extension of the N-terminus of hC3Nb1 might render it specific towards C3b. The inventors hence extended the N-terminus of hC3Nb1 by a Glu-Trp-Glu motif, which conferred specificity of the resulting EWE-hC3Nb1 toward C3b (Fig 3). The EWE-hC3Nb1 binds close to, but does not overlap with CCP2 of factor H.

### The complement system

The complement system is part of the innate immune system and plays an important role in protection against invading microorganisms and in maintenance of homoeostasis. The innate immune system is not adaptable and does not change over the course of an individual's lifetime. More than 50 proteins and protein fragments make up the complement system, including serum proteins, serosal proteins, and cell membrane bound receptors and regulatory proteins. A subset of the complement proteins circulates as inactive precursors (pro-proteins). When stimulated by one of several triggers, proteases in the system cleave specific proteins to initiate an amplifying cascade of further cleavages. The end-result of this activation cascade includes massive amplification of the response, enhanced phagocytosis and pathogen lysis, clearance of immune complexes and apoptotic cells, inflammation, stimulation of adaptive immune responses and assembly of the cell-killing membrane attack complex.

Uncontrolled activation or lack of proper regulation of complement is involved in a range of diseases and the present disclosure therefore provides means for pharmacological regulation of the complement cascade in order to ameliorate disease outcome.

More specifically, the complement system is activated by three different proteolytic pathways: The classical pathway (CP), the lectin pathway (LP) and the alternative pathway (AP). Activation of the complement system results in cleavage of the complement proteins C3 (all pathways) into C3a and C3b. After a certain threshold of C3b density is reached on the complement activating surface, activation of the terminal pathway (TP) results in cleavage of complement C5.

The CP is activated by the C1 complex formed by the pattern recognition molecule C1q and the serine proteases C1r and C1s. C1 recognition leads to activation of the C1 complex. C1 cleaves C4 into C4a and C4b, and C2 can now bind to C4b, and C2 is then cleaved by the C1 complex into C2a and C2b. C4bC2a is the CP C3 convertase that cleaves C3 into C3a and C3b.

The LP is initiated by recognition of carbohydrate or acetylated pathogen-associated molecular patterns (PAMPs) by mannose binding lectin (MBL), the collectin CL-LK and three ficolins, respectively. MBL, CL-LK and the ficolins are associated with MBL-associated serine proteases (MASP) -1 and -2 that are activated upon binding of MBL, CL-LK and ficolins to DAMPs and PAMPs. MASP-1 and MASP-2 activation triggers the same proteolytic cascade as the classical pathway and also leads to assembly of the CP C3 convertase.

The AP may be activated by a spontaneous hydrolysis of an internal thioester in C3, resulting in formation of C3(H₂O). C3(H2O) associates with the protease factor B (FB), which is cleaved by factor D into Bb and Ba. The complex between C3(H₂O) and Bb is the fluid phase AP C3 convertase that cleaves C3 into C3a and C3b. C3b reacts with nearby nucleophiles on surfaces and become covalently attached to these resulting in activator bound C3b. Such C3b associates with FB that is cleaved and the AP C3 convertase, C3bBb is formed. The AP C3 convertase cleaves C3 into C3b and C3a and more AP convertase is formed in an amplification loop. The activator bound C3b may also originate from C3 cleavage conducted by the CP C3 convertase, and indeed the AP C3 convertase strongly amplifies the initial C3 cleavage taking place in the CP and LP.

A fusion protein comprising a single domain antibody targeting C3b is capable of inhibiting interaction between C3b and the zymogen FB or the active Bb and thereby inhibit assembly of the C3bBb convertase complex and/or the activity of the C3bBb convertase. In a preferred embodiment, the single domain antibody is capable of inhibiting activation of the complement system.

In the context of the present disclosure individual complement proteins may be identified as the specific proteins shown in the sequence list further below, however the skilled person will appreciate that the present disclosure is not limited to the specific species or proteins disclosed herein.

### Single-domain antibodies

Naturally occurring human antibodies are heterotetramers. The antibodies comprised in the fusion proteins provided herein comprise an antigen binding site in a single polypeptide. The antibodies are therefore herein referred to as "single domain antibodies". Single domain antibodies are also known as nanobodies.

A single domain antibody is an antibody fragment consisting of a single monomeric variable antibody domain. Like a naturally occurring human antibody, it is able to bind selectively to a specific antigen. Single domain antibodies typically have molecular weights in the range of 12-15 kDa, i.e. much lower than common antibodies, ranging typically from 150 to 160 kDa. Single domain antibodies are also smaller than Fab fragments (-50 kDa) of heterotetrameric antibodies comprising one light chain and half a heavy chain.

Single domain antibodies can derive from antibodies found in nature, for example in camelids (V_{H}H) and cartilaginous fishes (V_{NAR}). New or Nurse Shark Antigen Receptor (NAR) protein exists as a dimer of two heavy chains with no associated light chains. Each chain is composed of one variable (V) and five constant domains. The NAR proteins thus constitute a single immunoglobulin variable-like domain. Single heavy-chain antibodies are also found in camelids, such as such as dromedaries, camels, llamas and alpacas, where the heavy chain has lost one of its constant domains and underwent modifications in the variable domain, both of which are structural elements necessary for the binding of light chains.

However, single domain antibodies can also be engineered by recombinant methods. One approach is to split the dimeric variable domains from common immunoglobulin G (IgG) from humans or mice into monomers. Single domains, which are derived from light chains, also bind specifically to target epitopes. Thus, the single domain antibody may be derived from any suitable organism.

Single domain camelid antibodies are equal to regular antibodies in terms of specificity. Single domain antibodies are easily isolated, for example by using phage panning procedures. The smaller size and single domain architecture make these antibodies easier to express as proteins in bacterial cells for large scale production, making them ideal for commercial exploitation. The single domain antibodies of the present disclosure are therefore preferably derived from camelid antibodies, preferably llama antibodies.

In one embodiment, the single domain antibody is further engineered to obtain specificity.

In one embodiment of the present disclosure, the single domain antibody comprises a N-terminal motif. In another embodiment of the present disclosure, the single domain antibody comprises or consists of the amino acid sequence of SEQ ID NO: 23 and a N-terminal motif. In one embodiment of the present disclosure, the N-terminal motif creates specificity towards C3b by sterically hindering binding to C3. In one embodiment of the present disclosure, the N-terminal motif is a protein, such as an IgG. The N-terminal motif may be a bulky N-terminal motif. In one embodiment of the present disclosure, the bulky N-terminal motif is an amino acid sequence consisting of up to 10 amino acids, such as 1, 2, 3, 4, 5, 6, 7, 8 or 9 amino acids. In another embodiment of the present disclosure, the bulky N-terminal motif is comprised of bulky amino acids, such as phenylalanine, tyrosine, tryptophan, arginine, histidine, lysine, tyrosine, glutamic acid and glutamine. In a further embodiment of the present disclosure, the bulky N-terminal motif is a 3 amino acid sequence consisting of the amino acids EWE.
In one embodiment of the present disclosure, the single domain antibody comprises or consists of an amino acid sequence having at least 80% sequence identity to the amino acid sequence according to SEQ ID NO: 1. In a preferred embodiment, the single domain antibody comprises or consists of an amino acid sequence having at least 81% sequence identity to the amino acid sequence SEQ ID NO: 1, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity. In a specific embodiment of the present disclosure, the single domain antibody comprises or consists of the amino acid sequence of SEQ ID NO: 1.

The single domain antibody of the present disclosure preferably comprise one or more complementary determining regions (CDRs). In particular, the CDRs may identify the specificity of the antibody and accordingly it is preferred that the antigen binding site comprises one or more CDRs, preferably at least 1, more preferably at least 2, yet more preferably 3 or more CDRs. In one specific embodiment, the single domain antibody comprises 3 CDRs. Given the high importance of the CDR regions in target binding it is generally considered that alterations in the sequence of the single domain antibodies are made outside of the CDRs. Thus, in one embodiment of the present disclosure, any sequence variance is outside the complementarity determining regions. The sequence of a CDR region can be annotated by different methods, once the sequence has been obtained. The exact boundaries of CDRs, and framework regions in the present invention have been determined using the Kabat method.

In one embodiment of the present disclosure, the single domain antibody comprises a CDR1 having the amino acid sequence of SEQ ID No: 2, a CDR2 having the amino acid sequence of SEQ ID No: 3 and a CDR3 having the amino acid sequence of SEQ ID No: 4.

In one embodiment of the present disclosure, the single domain antibody is capable of inhibiting the alternative pathway by preventing binding of factor B or fragments thereof to C3b. In another embodiment of the present disclosure, the single domain antibody is capable of hindering formation of the alternative pathway C3 convertase, C3bBb.

The strength of binding between receptors and their ligands, for example between an antibody and its antigen can be defined is its affinity towards an antigen. The affinity of an antibody can be defined in terms of the dissociation constant, K_{D}, which is an equilibrium constant that measures the propensity of a molecular complex to separate (dissociate) reversibly into the molecules forming the complex. The smaller the value of the K_{D} of an antibody, the higher affinity that the antibody binds to a target antigen. In one aspect, K_{D} is defined as the ratio k_{off} / kₒₙ, where k_{off} and kₒₙ are the rate constants for association and dissociation of the molecular complex. Preferably affinity is determined by calculating the dissociation constant K_{D} based on IC₅₀ values. Thus, the affinity is measured as an apparent affinity. It is preferred that the single domain antibodies as described herein bind with an affinity corresponding to a K_{D} of about 10⁻⁴ M or less, such as about 10⁻⁵ M or less, such as about 10⁻⁶ M or less, 10⁻⁷ M or less, such as about 10⁻⁸ M or less, such as about 10⁻⁹ M or less, about 10⁻¹⁰ M or less, or about 10⁻¹¹ M or even less, when measured based on apparent affinities based on EC₅₀ values (the effective concentration achieving 50% of maximal binding) in an ELISA assay. In one embodiment, the antibody binds with an affinity corresponding to a K_{D} of about 10⁻⁶ M or less, such as 10⁻⁶ to 10⁻¹⁸ M. K_{D} values for antibodies can be determined using methods in the art in view of the present disclosure. For example, the K_{D} of an antibody can be determined by using surface plasmon resonance, such as by using a biosensor system, e.g., a Biacore^{®} system, or by using bio-layer interferometry technology, such as an Octet RED96 system

The antibody may also or alternatively bind their target complement factor with an affinity corresponding to a K_{D} that is at least ten-fold lower, such as at least 100-fold lower, such as at least 1000-fold lower than its affinity for binding to a non-specific antigen (e.g., BSA or casein).

### Epitope

The antigen binding site of a single domain antibody is able to bind selectively to a specific antigen. The site on an antigen at which a single domain antibody binds is referred to as an epitope. An epitope can be a linear epitope or a conformational epitope. A linear epitope is an epitope that is recognized by antibodies by its linear sequence of amino acids, or primary structure. A conformational epitope, or a 3-dimensional epitope, has a specific three-dimensional shape and can be comprised of amino acids situated at different sites in a polypeptide chain or even on different polypeptide chains. An epitope can also be located on molecules other than polypeptides, such as saccharides and organic- and inorganic molecules.
One way of determining the epitope of which a protein binds to, is to determine its footprint.

The single domain antibodies comprised in the fusion proteins of the disclosure are capable of specifically binding to an epitope of C3b. In one embodiment of the present disclosure, the single domain antibody binds an epitope comprised in the amino acid sequence according to SEQ ID NO: 20. In another embodiment, of the present disclosure, the single domain antibody binds an epitope comprised in the MG7 domain of C3b and the N-terminus of the C3b alpha chain, such as an epitope comprised in the amino acid sequence according to SEQ ID NO: 20 and the N-terminus of SEQ ID NO: 19.

An antigen can comprise many different epitopes. A protein which is not an antibody can also bind to another protein, in some instances it will bind to an epitope with less specificity and less affinity. When two epitopes are situated in close proximity, a situation can occur where only one molecule, is able to bind it epitope, and hence compete for binding with the protein capable of binding said other epitope. In other situations two proteins/antibodies bind to the same epitope, and must compete for binding. In some aspects of the present disclosure, the epitope to which the single domain antibodies bind are described by their capability of competing for binding with other proteins. Thus, in one embodiment of the present disclosure, the single domain antibody competes for binding with the CCP1 domain of factor H, such as the amino acid sequence according to SEQ ID NO: 21.

In another embodiment of the present disclosure, the single domain antibody competes for binding with a protein capable of binding to an epitope consisting of the amino acids:
a) Glu759, Asn760, His918, His919, Phe920 of the amino acid sequence according to SEQ ID NO: 19;
b) Glu758, Glu759, Asn760, Arg855, Val916, His918, His919, Phe920 of the amino acid sequence according to SEQ ID NO: 19; or
c) Asp752, Asp754, Ile755, Ile756, Glu759, Asn760, His918, His919, Phe920 of the amino acid sequence according to SEQ ID NO: 19.

In a preferred embodiment of the present disclosure, the single domain antibody competes for binding with the single domain antibody as defined in SEQ ID NO: 1, i.e. it competes for binding with EWE-hC3Nb1.

In one embodiment of the present disclosure, the single domain antibody does not compete for binding to C3b with factor I, such as the amino acid sequence according to SEQ ID NO: 22. In another embodiment of the present disclosure, the single domain antibody does not compete for binding with the CCP2 or the CCP3 domain of factor H, such as the amino acid sequence according to SEQ ID NO: 11 or SEQ ID NO: 12.

### Polypeptide

The present disclosure provides both novel polypeptides and fusion proteins comprising the novel polypeptides. Polypeptides or proteins are complex, three-dimensional structures containing one or more long, folded polypeptide chains. Polypeptide chains are composed of a plurality of small chemical units called amino acids, which are connected in a N-terminal to C-terminal fashion via covalent peptide bonds. Naturally occurring amino acids have an L-configuration. Synthetic peptides can be prepared employing conventional synthetic methods, using L-amino acids, D-amino acids or various combinations of L- and D-amino acids. The term "peptide" describes a combination of two or more amino acids. 20 amino acids exist naturally and are typically designated using the following table:

| Amino acid | Letter code | |
|---|---|---|
| | 3 | 1 |
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The present disclosure provides in one aspect a polypeptide having a length of less than 220 amino acids, wherein the polypeptide comprises or consists of the amino acid sequence according to SEQ ID NO: 10 or a variant thereof having at least 80% sequence identity to the amino acid sequence according to SEQ ID NO: 10. In one embodiment of the present disclosure, the polypeptide is less than 240 amino acids, such as less than 235, 230, 225, 220, 215, 210, 205, 200, 195, 190, 185 amino acids. In one specific embodiment the polypeptide is 183 amino acids long. In a preferred embodiment, the polypeptide comprises or consists of an amino acid sequence having at least 81% sequence identity to the amino acid sequence SEQ ID NO: 10, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity.

In a further aspect of the present disclosure, the disclosure provides a polypeptide having a length of less than 350 amino acids, wherein the polypeptide comprises or consists of the amino acid sequence according to SEQ ID NO: 9 or a variant thereof having at least 80% sequence identity to the amino acid sequence according to SEQ ID NO: 9. In one embodiment of the present disclosure, the polypeptide is less than 380 amino acids, such as less than 375, 370, 365, 360, 355, 350, 345, 340, 335, 330, 325, 320 amino acids. In one specific embodiment the polypeptide is 319 amino acids long. In a preferred embodiment, the polypeptide comprises or consists of an amino acid sequence having at least 81% sequence identity to the amino acid sequence SEQ ID NO: 9, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity.

The polypeptides provided in the present disclosure are recombinant proteins derived from natural complement proteins. In one embodiment of the present disclosure, the polypeptide is derived from the complement co-factor protein factor H. In certain embodiments of the present disclosure, the polypeptide comprises specific domains from factor H, such as complement control protein (CCP). The CCP domain is an evolutionarily conserved protein domain. It is also known as a sushi domain or short consensus repeats (SCR). The name derives from the visual similarity of the domain to nigiri sushi when the primary structure is drawn showing the loops created by the disulfide bonds. Sushi domains exist in a wide variety of complement and adhesion proteins. The structure is known for this domain; it is based on a beta-sandwich arrangement - one face made up of three β-strands hydrogen-bonded to form a triple-stranded region at its center, and the other face formed from two separate β-strands. In some aspects of the disclosure, the polypeptides provided are minimized versions of factor H. Such a prior art protein, consisting of CCP domains 1-4 and 19-20, has previously been referred to as a mini-FH. The minimized versions of the present disclosure may preferably be provided in versions not comprising CCP1 (SEQ ID NO: 21). Thus, in specific embodiments, the present disclosure provides µH (micro-H) and nH (nano-H), wherein µH comprises CCP domains 2-4 and 19-20 and nH comprises CCP domains 2-4.

In one embodiment of the present disclosure, the polypeptide comprises or consists of:
a) the amino acid sequence according to SEQ ID NO: 10 or a variant thereof having at least 80% sequence identity to the amino acid sequence according to SEQ ID NO: 10;
b) the amino acid sequence according to SEQ ID NO: 18 or a variant thereof having at least 80% sequence identity to the amino acid sequence according to SEQ ID NO: 18; and
c) a second linker connecting the amino acid sequence according to SEQ ID NO: 10 to the amino acid sequence according to SEQ ID NO: 18, wherein the second linker may consist of the amino acid sequence according to SEQ ID NO: 17.

In one embodiment of the present disclosure, the polypeptide comprises or consists of the amino acid sequence according to SEQ ID NO: 9 or a variant thereof having at least 80% sequence identity to the amino acid sequence according to SEQ ID NO: 9. In a preferred embodiment, the polypeptide comprises or consists of an amino acid sequence having at least 81% sequence identity to the amino acid sequence SEQ ID NO: 9, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity.

In one embodiment of the present disclosure, the polypeptide is capable of recruiting factor I, such as recruiting the amino acid sequence according to SEQ ID NO: 22. In one embodiment of the present disclosure, the polypeptide is capable of activating degradation of C3b by recruiting factor I, such as recruiting the amino acid sequence according to SEQ ID NO: 22. In one embodiment of the present disclosure, the polypeptide is a co-factor for factor I, such as the co-factor according to the amino acid sequence according to SEQ ID NO: 22. In one embodiment of the present disclosure, the polypeptide does not comprise the amino acid sequence according to SEQ ID NO: 21, or a variant thereof having at least 80% sequence identity to the amino acid sequence according to SEQ ID NO: 21.

### Fusion proteins

The present disclosure also provides fusion proteins comprising a single domain antibody and a polypeptide as described above. Such fusion protein can be assembled by methods known to the person skilled in the art. Preferably, the fusion protein is recombinantly designed by fusing gene sequences in vitro. A fusion protein provided herein may consist of a single domain antibody and a polypeptide. In some embodiments, the fusion protein further comprises a linker connecting the two.

In one embodiment of the present disclosure, the fusion protein comprises or consists of an amino acid sequence having at least 80% sequence identity to the amino acid sequence according to SEQ ID NO: 7, or a variant thereof having at least 80% sequence identity to the amino acid sequence according to SEQ ID NO: 7. In a preferred embodiment, the fusion protein comprises or consists of an amino acid sequence having at least 81% sequence identity to the amino acid sequence SEQ ID NO: 7, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity. In one embodiment of the present disclosure, the fusion protein comprises or consists of the amino acid sequence of SEQ ID NO: 7.

In another embodiment of the present disclosure, the fusion protein comprises or consists of an amino acid sequence having at least 80% sequence identity to the amino acid sequence according to SEQ ID NO: 5. In a preferred embodiment, the fusion protein comprises or consists of an amino acid sequence having at least 81% sequence identity to the amino acid sequence SEQ ID NO: 5, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity. In one embodiment of the present disclosure, the fusion protein comprises or consists of the amino acid sequence of SEQ ID NO: 5.

In further embodiments of the present disclosure, the polypeptide comprised in the fusion protein is any recombinant variant of the complement protein factor H, wherein the recombinant variant of the complement protein factor H does not comprise CCP1, such as the amino acid sequence according to SEQ ID NO: 21, or a variant thereof having at least 80% sequence identity to the amino acid sequence according to SEQ ID NO: 21. The full length version of the human orthologue of factor H is a 1231 amino acid polypeptide and the CCP1 domain is a 64 amino acid polypeptide. The CCP2 domain starts at position 83 of the human factor H, thus in one embodiment of the present disclosure, the polypeptide has a maximal length of 1148 amino acids. The average length of the CCP domains of human factor H is 59 amino acids, and thus various recombinant variants of the complement protein factor H can be made where one or more CCP domains are removed. It is preferred that at least one of the domains CCP2, CCP3 or CCP4 are retained in the polypeptide, such as at least CCP2-3 or CCP3-4. In a preferred embodiment of the disclosure, the domains CCP2-4 are retained in the recombinant variant of the complement protein factor H. When one or more domains are removed, one or more linkers may be added to provide the correct flexibility in the polypeptide chain. Since essentially any of the CCP domains can be retained in the polypeptide, the polypeptide can have various lengths of less than 1148 amino acids, such as less than 1140 amino acids, such as less than 1130 amino acids, such as less than 1120 amino acids, such as less than 1110 amino acids, such as less than 1100 amino acids, such as less than 1090 amino acids, such as less than 1080 amino acids, such as less than 1070 amino acids, such as less than 1060 amino acids, such as less than 1050 amino acids, such as less than 1040 amino acids, such as less than 1030 amino acids, such as less than 1020 amino acids, such as less than 1010 amino acids, such as less than 1000 amino acids, such as less than 990 amino acids, such as less than 980 amino acids, such as less than 970 amino acids, such as less than 960 amino acids, such as less than 950 amino acids, such as less than 940 amino acids, such as less than 930 amino acids, such as less than 920 amino acids, such as less than 910 amino acids, such as less than 900 amino acids, such as less than 890 amino acids, such as less than 880 amino acids, such as less than 870 amino acids, such as less than 860 amino acids, such as less than 850 amino acids, such as less than 840 amino acids, such as less than 830 amino acids, such as less than 820 amino acids, such as less than 810 amino acids, such as less than 800 amino acids, such as less than 790 amino acids, such as less than 780 amino acids, such as less than 770 amino acids, such as less than 760 amino acids, such as less than 750 amino acids, such as less than 740 amino acids, such as less than 730 amino acids, such as less than 720 amino acids, such as less than 710 amino acids, such as less than 700 amino acids, such as less than 690 amino acids, such as less than 680 amino acids, such as less than 670 amino acids, such as less than 660 amino acids, such as less than 650 amino acids, such as less than 640 amino acids, such as less than 630 amino acids, such as less than 620 amino acids, such as less than 610 amino acids, such as less than 600 amino acids, such as less than 590 amino acids, such as less than 580 amino acids, such as less than 570 amino acids, such as less than 560 amino acids, such as less than 550 amino acids, such as less than 540 amino acids, such as less than 530 amino acids, such as less than 520 amino acids, such as less than 510 amino acids, such as less than 500 amino acids, such as less than 490 amino acids, such as less than 480 amino acids, such as less than 470 amino acids, such as less than 460 amino acids, such as less than 450 amino acids, such as less than 440 amino acids, such as less than 430 amino acids, such as less than 420 amino acids, such as less than 410 amino acids, such as less than 400 amino acids, such as less than 390 amino acids or such as less than 380 amino acids.

In a preferred embodiment, the polypeptide is a polypeptide having a length of less than 350 amino acids, wherein the polypeptide comprises or consists of the amino acid sequence according to SEQ ID NO: 9 or a variant thereof having at least 80% sequence identity to the amino acid sequence according to SEQ ID NO: 9. In one embodiment of the present disclosure, the polypeptide is less than 380 amino acids, such as less than 375, 370, 365, 360, 355, 350, 345, 340, 335, 330, 325 or 320 amino acids. In one specific embodiment the polypeptide is 319 amino acids long. In a preferred embodiment, the polypeptide comprises or consists of an amino acid sequence having at least 81% sequence identity to the amino acid sequence SEQ ID NO: 9, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity.

In another preferred embodiment, the polypeptide is a polypeptide having a length of less than 220 amino acids, wherein the polypeptide comprises or consists of the amino acid sequence according to SEQ ID NO: 10 or a variant thereof having at least 80% sequence identity to the amino acid sequence according to SEQ ID NO: 10. In one embodiment of the present disclosure, the polypeptide is less than 240 amino acids, such as less than 235, 230, 225, 220, 215, 210, 205, 200, 195, 190 or 185 amino acids. In one specific embodiment the polypeptide is 183 amino acids long. In a preferred embodiment, the polypeptide comprises or consists of an amino acid sequence having at least 81% sequence identity to the amino acid sequence SEQ ID NO: 10, such as at least 82 %, such as at least 83 %, such as at least 84 %, such as at least 85 %, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98% or such as at least 99% sequence identity.

When the recombinant fusion proteins of the disclosure comprise a C3b specific single domain antibody and a recombinant polypeptide described above, the resulting fusion protein has the surprising advantage of combining an inhibition of the alternative pathway as well as removing surface bound C3b molecules. In one embodiment of the present disclosure, the fusion protein is capable of inhibiting the alternative pathway by preventing binding of factor B or fragments thereof to C3b and furthermore is capable of recruiting factor I, such as recruiting the amino acid sequence according to SEQ ID NO: 22. In one embodiment of the present disclosure, the fusion protein is capable of hindering formation of the alternative pathway C3 convertase, C3bBb and furthermore is capable of recruiting factor I, such as recruiting the amino acid sequence according to SEQ ID NO: 22. In one embodiment of the present disclosure, the fusion protein is capable of inhibiting the alternative pathway by preventing binding of factor B or fragments thereof to C3b and furthermore is capable of activating degradation of C3b by recruiting factor I. In one embodiment of the present disclosure, the fusion protein is capable of inhibiting the alternative pathway by preventing binding of factor B or fragments thereof to C3b and furthermore is a co-factor for factor I, such as the co-factor according to the amino acid sequence according to SEQ ID NO: 22. In one embodiment of the present disclosure, the fusion protein is capable of hindering formation of the alternative pathway C3 convertase, C3bBb and furthermore is capable of activating degradation of C3b by recruiting factor I, such as recruiting the amino acid sequence according to SEQ ID NO: 22. In one embodiment of the present disclosure, the fusion protein is capable of hindering formation of the alternative pathway C3 convertase, C3bBb and furthermore is a co-factor for factor I, such as the co-factor according to the amino acid sequence according to SEQ ID NO: 22.

### Antibody modifications

The single domain antibodies disclosed herein above, may in preferred embodiments comprise modifications, which improve the function and/or usability of the antibody. For example, it is not always desirable to use non-human antibodies for human therapy, and accordingly the single domain antibodies provided herein may be humanized antibodies.

The single domain antibody according to the disclosure may be a humanized single domain antibody. A human antibody as used herein is an antibody, which is obtained from a system using human immunoglobulin sequences. Human antibodies may for example be antibodies isolated from an animal (e.g., a mouse) that is transgenic or transchromosomal for human immunoglobulin genes or a hybridoma prepared therefrom. Human antibodies may also be isolated from a host cell transformed to express the antibody, e.g., from a transfectoma. Human antibodies may also be isolated from a recombinant, combinatorial human antibody library or directly cloned from human B cells.

Human antibodies have variable and constant regions derived from human germline immunoglobulin sequences. In certain embodiments, however, such recombinant human antibodies can be subjected to *in vitro* mutagenesis or *in vivo* somatic mutagenesis and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

A human antibody is preferably at least 90%, more preferably at least 95%, even more preferably at least 96%, 97%, 98%, or 99% identical in amino acid sequence to the amino acid sequence encoded by a wild type human immunoglobulin gene.

Said transgenic of transchromosomal animal may contain a human immunoglobulin gene miniloci that encodes unrearranged human heavy (µ and/or γ) and κ light chain immunoglobulin sequences. Furthermore, the animal may contain one or more mutations that inactivate the endogenous heavy and light chain loci.

The single domain antibody of the disclosure may be a chimeric antibody, i.e. an antibody comprising regions derived from different species. The chimeric antibody may for example comprise variable regions from one species of animal and constant regions from another species of animal. For example, a chimeric antibody can be an antibody having variable regions, which derive from a llama monoclonal antibody and constant regions, which are human. Such antibodies may also be referred to as humanized antibodies. The single domain antibodies can advantageously be humanized in order to prevent immunological reactions of the human organism against the antibody.

Thus, the single domain antibodies provided herein may be a humanized antibody, which is encoded partly by sequences obtained from human germline immunoglobulin sequences and partly from other sequences. Said other sequences are preferably germline immunoglobulines from other species, which produce single domain antibodies, most preferably from camelidae species, such as llama. In particular, a humanized antibody may be an antibody in which the antigen binding site is derived from an immunoglobulin from llama, whereas some or all of the remaining immunoglobulin-derived parts of the molecule is derived from a human immunoglobulin. The antigen binding site from said llama may for example consist of a complete V_{H}H or one or more CDRs grafted onto appropriate human framework regions. Thus, in a humanized antibody, the CDRs can be from camelids or cartilaginous fishes, preferably llama, and the other regions of the antibody are of human origin.

In other embodiments, the single domain antibodies are modified by codon optimization or other modifications introduced in order to enhance the function and/or usability of the antibody.

### Linker

The linkers of the present disclosure are designed to be non-immunogenic and are preferably also flexible linkers. Preferably, the length of the linker is from 15 to 25 amino acids to secure flexibility. In another preferred embodiment, the length of the linker is from 8 to 25 amino acids, such as from 8 to 20 amino acids, such as from 8 to 15 amino acids, for example 8 to 12 amino acids or such as for example from 10 to 15 amino acids. The linker can comprise 10, 11, 12, 13, 14, 15, 16, 17, 18, 19, 20, 21, 22, 23, 24 or even 25 amino acids. In a particular embodiment, the length of the linker is 20 amino acids.

The linker is preferably a serine-glycine (GS) linker, such as a flexible GGGGS linker, such as GGGSS, GGGSG, GGGGS or multiple variants thereof such as GGGGSGGGGS or (GGGGS)m, (GGGSS)m, (GGGSG)m, where m is an integer from 1 to 5, from 1 to 4 or from 1 to 3. In a preferred embodiment m is 4.

In a preferred embodiment the serine-glycine linker further comprises at least one leucine (L), such as at least 2 or at least 3 leucines. The serine-glycine linker may for example comprise 1, 2, 3, 4, 5, 6, 7 leucines. Preferably, the serine-glycine linker comprises 2 leucine or 4 leucines.

In one embodiment the linker comprises or consists of the sequence LGGGS, GLGGS, GGLGS, GGGLS or GGGGL. In another embodiment the linker comprises or consists of the sequence LGGSG, GLGSG, GGLSG, GGGLG or GGGSL. In yet another embodiment the linker comprises or consists of the sequence LGGSS, GLGSS, GGLSS, GGGLS or GGGSL.

In yet another embodiment the linker comprises or consists of the sequence LGLGS, GLGLS, GLLGS, LGGLS or GLGGL. In another embodiment the linker comprises or consists of the sequence LGLSG, GLLSG, GGLSL, GGLLG or GLGSL. In yet another embodiment the linker comprises or consists of the sequence LGLSS, GLGLS, GGLLS, GLGSL or GLGSL.

In another embodiment of the present invention the serine-glycine linker has a length of 20 amino acids and comprises 2 leucine or 4 leucines.

In one embodiment the linker comprises or consists of the sequence LGGGSGGGGS, GLGGSGGGGS, GGLGSGGGGS, GGGLSGGGGS or GGGGLGGGGS. In another embodiment the linker comprises or consists of the sequence LGGSG GGGSG, GLGSGGGGSG, GGLSGGGGSG, GGGLGGGGSG or GGGSLGGGSG. In yet another embodiment the linker comprises or consists of the sequence LGGSSGGGSS, GLGSSGGGSS, GGLSSGGGSS, GGGLSGGGSS or GGGSLGGGSS.

In a further embodiment the linker comprises or consists of the sequence LGGGSLGGGS, GLGGSGLGGS, GGLGSGGLGS, GGGLSGGGLS or GGGGLGGGGL. In another embodiment the linker comprises or consists of the sequence LGGSGLGGSG, GLGSGGLGSG, GGLSGGGLSG, GGGLGGGGLG or GGGSLGGGSL. In yet another embodiment the linker comprises or consists of the sequence LGGSSLGGSS, GLGSSGLGSS, GGLSSGGLSS, GGGLSGGGLS or GGGSLGGGSL.

Alternative linkers may be selected from the group consisting of GSAT linkers and SEG linkers, or multiple variants thereof.

In one embodiment of the present disclosure, the first linker is a glycine-serine (GS) linker. In one embodiment of the present disclosure, the first linker consists of between 15 and 25 amino acids. In one embodiment of the present disclosure, the first linker is between 45 Å and 130 Å long, such as between 55 Å and 120 Å, between 60 Å and 80 Å or between 65 and 70 Å long. In one embodiment of the present disclosure, the first linker is as defined in SEQ ID NO: 6.
In one embodiment of the present disclosure, the second linker is as defined in SEQ ID NO: 17.

### Nucleic acid and vectors

The present disclosure further provides nucleic acids and vectors encoding the polypeptides and/or fusion proteins of the disclosure. In one embodiment, the nucleic acid is a polynucleotide. The polynucleotide may comprise a DNA nucleotide sequence or a RNA nucleotide sequence, such as genomic DNA, cDNA, and RNA sequences, either double stranded or single stranded. It is preferred that the polynucleotide is optimized to the species to express the polypeptide according to the invention, i.e. it is preferred that the polynucleotide sequence is human codon optimized.

Thus in one aspect, the disclosure provides a nucleic acid encoding the fusion protein or the polypeptide disclosed herein.

Furthermore, the invention relates to a vector comprising a nucleotide sequence as defined above. It is preferred that the vector allows for easy exchange of the nucleic acids.

Thus, in a further aspect, the disclosure provides a vector comprising the nucleic acid. In an even further aspect, the disclosure provides a method of preparing the vector, the method comprising:
e) introducing the vector into a cell;
f) culturing the cell;
g) collecting and purifying the vector from the cell; and
h) optionally, lysing the cell to release the vector from the cell.

### Host cells and methods of manufacture

The present disclosure also provide host cells comprising nucleic acids and vectors comprising the polypeptides and/or fusion proteins of the disclosure as well as methods of manufacturing the polypeptides and/or fusion proteins of the disclosure. Suitable host cells include prokaryotes, yeast, insect or higher eukaryotic cells.

In one aspect, the disclosure provides a host cell comprising the nucleic acid or the vector described herein.
In a further aspect, the disclosure provides a method of preparing the fusion protein or the polypeptide of the disclosure, the method comprising:
e) introducing the nucleic acid or the vector encoding the polypeptides and/or fusion proteins of the disclosure into a host cell;
f) culturing the cell;
g) obtaining and optionally purifying the fusion protein or polypeptide from the supernatant; and
h) optionally, lysing the cell prior to purifying the fusion protein or the polypeptide.

### Pharmaceutical composition and methods of treatment

A pharmaceutical composition is a composition comprising one or more substances that have medicinal properties, together with a pharmaceutical acceptable carrier. Details of pharmaceutical compositions are provided herein below.

One aspect of the present invention relates to a pharmaceutical composition comprising one or more of the fusion proteins or the polypeptides provided herein.

The fusion proteins or the polypeptides can be formulated as pharmaceutical compositions and administered to a mammalian host, such as a human patient in a variety of forms adapted to the chosen route of administration. In another approach, the fusion proteins or the polypeptides may be administered as DNA, e.g. by use of Adeno-associated viruses (AAV) and then expressed from the vector.

In one aspect the invention relates to a pharmaceutical composition comprising the fusion proteins or the polypeptides disclosed herein, optionally comprising a pharmaceutically acceptable carrier.

In a further aspect, the invention relates to a method of treating a disorder associated with complement activation, the method comprising administering a therapeutically effective amount of the fusion proteins, the polypeptides or the pharmaceutical composition disclosed herein to a subject in need thereof.

In one embodiment the present disclosure provides the fusion protein, the polypeptide or the pharmaceutical composition for use as a medicament.

In one embodiment the present disclosure provides the fusion protein, the polypeptide or the pharmaceutical composition for use in the treatment of a disorder associated with complement activation, such as a disorder selected from the group consisting of ocular diseases, neurological diseases, autoimmune and inflammatory disorders, cancers and infectious diseases.

In one preferred embodiment, the fusion protein, the polypeptide or the pharmaceutical composition are provided for use in treatment of ocular diseases. Ocular diseases may be selected from the group consisting of Occular, acute closed angle glaucoma, all stages of age-related macular degeneration (wet and dry), Behcet's retinopathy, Central Retinal Vein Occlusion (CRVO), choroidal neovascularization (CNV), Chronic open-angle glaucoma, corneal neovascularization, diabetic and other ischemia-related retinopathies, diabetic macular edema, diabetic retinopathy, endophthalmitis, Geographic atrophy, histoplasmosis of the eye, ischemia-related retinopathy, ischemic optic neuropathy, Leber's hereditary optic neuropathy, macular degenerative diseases, Neuromyelitis Optica (NMO), pathological myopia, Purtscher retinopathy, retinal neovascularization, Sjogren's dry eye disease Uveitis.

In another preferred embodiment, the fusion protein, the polypeptide or the pharmaceutical composition are provided for use in treatment of neurological diseases, such as diseases selected from the group consisting of Alzheimer's disease, schizophrenia, amyotrophic lateral sclerosis, Guillain-Barre syndrome, Huntington's disease, multiple sclerosis and Parkinson's disease.

In yet another preferred embodiment, the fusion protein, the polypeptide or the pharmaceutical composition are provided for use in treatment of autoimmune and inflammatory disorders, such as disorders selected from ANCA vasculitis, anti-nuclear cytoplasmic antigen-associated pauci-immune vasculitis (Wegener's syndrome), anti-phospholipid syndrome (APS), astma, atypical hemolytic uremic syndrome (aHUS), autoimmune hemolytic anemias, Bullous Pemphigoid, C3 glomerulonephritis, Coeliac disease, Cold agglutinin disease, Crohn's disease, cryoglobulemia, dense deposit disease, dermatomyositis, diabetes, Diabetes mellitus type 1, epidermolysis bullosa, Hashimoto's thyroiditis, hyperacute rejection. hypocomplementemic urticarial vasculitis (HUV), IgA nephropathy, intestinal and renal ischemia-reperfusion (IR) injury, lupus nephritis and resultant glomerulonephritis and vasculitis, Myasthenia Gravis, myositis, optic neuritis, paraneoplastic syndromes, paroxysomal nocturnal hemoglobinuria (PNH), pemphigus including Pemphigus vulgaris, polyarteritis nodosa, polymyalgia rheumatic, post-traumatic shock, acute renal failure, remote tissue injury after ischemia and reperfusion retinal vasculitis, rheumatoid arthritis (RA), sarcoidosis, sepsis, stroke, systemic lupus erythematosus (SLE), temporal arteritis, traumatic brain and spinal cord injury, type II membranoproliferative glomerulonephritis, vasculitis disease, vitiligo, acute respiratory distress syndrome (ARDS), chronic occlusive pulmonary distress syndrome (COPD), atherosclerosis, cardioplegia-induced coronary endothelial dysfunction, spontaneous and recurrent pregnancy loss, Addison's disease.

In another preferred embodiment, the fusion protein, the polypeptide or the pharmaceutical composition are provided for use in treatment of cancers such as carcinomas, sarcomas, lymphomas, leukaemia's, germ cell tumor, blastoma.

In a further aspect, the disclosure relates to a method of modulating the activity of the complement system, the method comprising: administering to an individual in need thereof a therapeutically effective amount of the fusion protein, the polypeptide or the pharmaceutical composition of the disclosure, thereby modulating the activity of the complement system in the subject in need thereof.

In other embodiments, the fusion protein or the polypeptide according to the invention is administered in an amount sufficient to modulate, i.e. inhibit or increase the activity of the classical pathway, the lectin pathway and/or the alternative pathway in said individual.

The individual may suffer from a clinical or physiological disorder or condition associated with increased activity of the complement system. In another embodiment, the individual may suffer from a disorder or clinical or physiological condition, which is treatable by increasing and/or recruiting the activity of the complement system.

### Administration forms

As described herein above, the fusion protein or the polypeptide composition provided herein can be used for medical/therapeutic treatment. In these aspects, the fusion proteins or the polypeptides are administered to a subject in need of treatment, and any suitable route of administration may be chosen, depending on the circumstances. Preferred routes of administration are described herein below.

### Systemic treatment

The main route of administration is parenteral in order to introduce fusion proteins, polypeptides or pharmaceutical compositions according to the invention into the blood stream to ultimately target the sites of desired action.

Appropriate dosage forms for such administration may be prepared by conventional techniques.

### Parenteral administration

Parenteral administration is any administration route not being the oral/enteral route whereby the medicament avoids first-pass degradation in the liver. Accordingly, parenteral administration includes any injections and infusions, for example bolus injection or continuous infusion, such as intravenous administration, intramuscular administration, subcutaneous administration. Furthermore, parenteral administration includes inhalations and topical administration.

The subcutaneous and intramuscular forms of parenteral administration are generally preferred.

### Pharmaceutical composition

Whilst it is possible for the fusion proteins or the polypeptides provided herein to be administered in raw form, it is preferred to present them in the form of a pharmaceutical composition. Accordingly, the present invention further provides a pharmaceutical composition, which comprises a fusion protein or a polypeptide of the present invention and a pharmaceutically acceptable carrier therefore. The pharmaceutical compositions may be prepared by conventional techniques, e.g. as described in Remington: The Science and Practice of Pharmacy 2005, Lippincott, Williams & Wilkins.

### Dosages and dosing regimes

The dosage requirements will vary with particular composition employed, the route of administration and the subject being treated. It will also be recognized by one of skill in the art that the optimal quantity and spacing of individual dosages of the fusion proteins or the polypeptides will be determined by the nature and extent of the condition being treated, the form, route and site of administration, and the particular patient being treated.

The fusion proteins or the polypeptides may be provided and/or administered as a unit dosage form. The term "unit dosage form" as used herein refers to physically discrete units suitable as unitary dosages for human and animal subjects, each unit containing a predetermined quantity of the fusion proteins or the polypeptides, alone or in combination with other agents, calculated in an amount sufficient to produce the desired effect in association with a pharmaceutically acceptable diluent, carrier, or vehicle. The specifications for the unit dosage forms of the present invention depend on the particular fusion proteins or the polypeptides employed and the effect to be achieved, as well as the pharmacodynamics associated with each antibody in the host. The dose administered should be an "effective amount" or an amount necessary to achieve an "effective level" in the individual patient.

When the "effective level" is used as the preferred endpoint for dosing, the actual dose and schedule can vary, depending on inter-individual differences in pharmacokinetics, drug distribution, and metabolism. The "effective level" can be defined, for example, as the blood or tissue level desired in the patient that corresponds to a concentration of one or more fusion proteins or polypeptides according to the invention.

The fusion proteins or the polypeptides of the present invention may be formulated in a wide variety of compositions for parenteral administration.

### Sequence identity

Sequence identity may be determined as follows: A high level of sequence identity indicates likelihood that the first sequence is derived from the second sequence. Amino acid sequence identity requires identical amino acid sequences between two aligned sequences. Thus, a candidate sequence sharing 70% amino acid identity with a reference sequence requires that, following alignment, 70% of the amino acids in the candidate sequence are identical to the corresponding amino acids in the reference sequence. Identity may be determined by aid of computer analysis, such as, without limitations, the ClustalW computer alignment program, and the default parameters suggested therein. Using this program with its default settings, the mature (bioactive) part of a query and a reference polypeptide are aligned. The number of fully conserved residues is counted and divided by the length of the reference polypeptide. In doing so, any tags or fusion protein sequences, which form part of the query sequence, are disregarded in the alignment and subsequent determination of sequence identity.

The ClustalW algorithm may similarly be used to align nucleotide sequences. Sequence identities may be calculated in a similar way as indicated for amino acid sequences.

Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the FASTA sequence alignment software package. Align calculates sequence identities based on a global alignment. Align0 does not penalise to gaps in the end of the sequences. When utilizing the ALIGN og Align0 program for comparing amino acid sequences, a BLOSUM50 substitution matrix with gap opening/extension penalties of ― 12/-2 is preferably used.

### Items

1) A fusion protein comprising:
   a) a single domain antibody that binds to C3b;
   b) a polypeptide comprising or consisting of the amino acid sequence according to SEQ ID NO: 10, or a variant thereof having at least 80% sequence identity to the amino acid sequence according to SEQ ID NO: 10; and
   c) a first linker.
2) The fusion protein according to item 1, wherein the single domain antibody binds an epitope comprised in the MG7 domain of C3b and the N-terminus of the C3b alpha chain, such as an epitope comprised in the amino acid sequence according to SEQ ID NO: 20 and the N-terminus of SEQ ID NO: 19.
3) The fusion protein according to any of the preceding items, wherein the single domain antibody competes for binding with the CCP1 domain of factor H, such as wherein the single domain antibody competes for binding with the amino acid sequence according to SEQ ID NO: 21.
4) The fusion protein according to any of the preceding items, wherein the single domain antibody competes for binding with the single domain antibody as defined in SEQ ID NO: 1.
5) The fusion protein according to any of the preceding items, wherein the single domain antibody competes for binding with a protein, which binds to an epitope consisting of the amino acids:
   a) Glu759, Asn760, His918, His919, Phe920 of the amino acid sequence according to SEQ ID NO: 19;
   b) Glu758, Glu759, Asn760, Arg855, Val916, His918, His919, Phe920 of the amino acid sequence according to SEQ ID NO: 19; or
   c) Asp752, Asp754, Ile755, Ile756, Glu759, Asn760, His918, His919, Phe920 of the amino acid sequence according to SEQ ID NO: 19.
6) The fusion protein according to any of the preceding items, wherein the single domain antibody does not compete for binding to C3b with factor I, such as wherein the single domain antibody does not compete for binding with the amino acid sequence according to SEQ ID NO: 22.
7) The fusion protein according to any of the preceding items, wherein the single domain antibody does not compete for binding with the CCP2 or the CCP3 domain of factor H, such as wherein the single domain antibody does not compete for binding with the amino acid sequence according to SEQ ID NO: 11 or SEQ ID NO: 12.
8) The fusion protein according to any of the preceding items, wherein the single domain antibody comprises a CDR1 having the amino acid sequence of SEQ ID NO: 2, a CDR2 having the amino acid sequence of SEQ ID NO: 3 and a CDR3 having the amino acid sequence of SEQ ID NO: 4.
9) The fusion protein according to any of the preceding items, wherein the single domain antibody comprises a N-terminal motif, such as a bulky N-terminal motif.
10) The fusion protein according to item 9, wherein the N-terminal motif creates specificity towards C3b by sterically hindering binding to C3.
11) The fusion protein according to any of items 9 or 10, wherein the N-terminal motif is a protein, such as an IgG.
12) The fusion protein according to any of items 9 or 10, wherein the bulky N-terminal motif is an amino acid sequence consisting of up to 10 amino acids, such as 1, 2, 3, 4, 5, 6, 7, 8, 9 or 10 amino acids.
13) The fusion protein according to any of items 9-10 or item 12, wherein the bulky N-terminal motif is comprised of one or more, such as two or more, such as three or more bulky amino acids, such as an amino acid selected from phenylalanine, tyrosine, tryptophan, arginine, histidine, lysine, tyrosine, glutamic acid and glutamine.
14) The fusion protein according to any of items 9-10 or 12-13, wherein the bulky N-terminal motif is a 3 amino acid sequence comprising or consisting of EWE.
15) The fusion protein according to any of the preceding items, wherein the single domain antibody comprises or consists of an amino acid sequence having at least 80% sequence identity to the amino acid sequence according to SEQ ID NO: 1.
16) The fusion protein according to any of the preceding items, wherein any sequence variance is outside the CDRs.
17) The fusion protein according to any of the preceding items, wherein the single domain antibody comprises or consists of the amino acid sequence of SEQ ID NO: 1, or is a humanized version of the single domain antibody having the amino acid sequence of SEQ ID NO: 1.
18) The fusion protein according to any of the preceding items, wherein the single domain antibody inhibits the alternative pathway by preventing binding of factor B or fragments thereof to C3b.
19) The fusion protein according to any of the preceding items, wherein the single domain antibody hinders formation of the alternative pathway C3 convertase, C3bBb.
20) The fusion protein according to any of the preceding items, wherein the first linker is a glycine-serine (GS) linker.
21) The fusion protein according to any of the preceding items, wherein the first linker consists of between 15 and 25 amino acids.
22) The fusion protein according to any of the preceding items, wherein the first linker is between 45 Å and 130 Å long, such as between 55 Å and 120 Å, between 60 Å and 80 Å or between 65 and 70 Å long.
23) The fusion protein according to any of the preceding items, wherein the first linker comprises or consists of an amino acid sequence according to SEQ ID NO: 6.
24) The fusion protein according to any of the preceding items, wherein the polypeptide has a length of less than 220 amino acids.
25) The fusion protein according to any of the preceding items, wherein the polypeptide comprises or consists of:
   a) the amino acid sequence according to SEQ ID NO: 10 or a variant thereof having at least 80% sequence identity to the amino acid sequence according to SEQ ID NO: 10;
   b) the amino acid sequence according to SEQ ID NO: 18 or a variant thereof having at least 80% sequence identity to the amino acid sequence according to SEQ ID NO: 18; and
   c) a second linker connecting the amino acid sequence according to SEQ ID NO: 10 to the amino acid sequence according to SEQ ID NO: 18, such as wherein the second linker comprises or consist of the amino acid sequence according to SEQ ID NO: 17.
26) The fusion protein according to item 25, wherein the polypeptide has a length of less than 350 amino acids.
27) The fusion protein according to item 25, wherein the polypeptide has a length of less than 1148 amino acids.
28) The fusion protein according to any of the preceding items, wherein the second linker consists of the amino acid sequence according to SEQ ID NO: 17.
29) The fusion protein according to any of the preceding items, wherein the polypeptide comprises or consists of the amino acid sequence according to SEQ ID NO: 9 or a variant thereof having at least 80% sequence identity to the amino acid sequence according to SEQ ID NO: 9.
30) The fusion protein according to any of the preceding items, wherein the polypeptide recruits factor I, such as wherein the polypeptide recruits the amino acid sequence according to SEQ ID NO: 22.
31) The fusion protein according to any of the preceding items, wherein the polypeptide activates degradation of C3b by recruiting factor I.
32) The fusion protein according to any of the preceding items, wherein the polypeptide is a co-factor for factor I, such as the co-factor according to the amino acid sequence according to SEQ ID NO: 22.
33) The fusion protein according to any of the preceding items, wherein the polypeptide does not comprise the amino acid sequence according to SEQ ID NO: 21, or a variant thereof having at least 80% sequence identity to the amino acid sequence according to SEQ ID NO: 21.
34) The fusion protein according to any of the preceding items, wherein the fusion protein comprises or consists of an amino acid sequence having the amino acid sequence according to SEQ ID NO: 7, or a variant thereof having at least 80% sequence identity to the amino acid sequence according to SEQ ID NO: 7.
35) The fusion protein according to any of the preceding items, wherein the fusion protein comprises or consists of the amino acid sequence of SEQ ID NO: 7.
36) The fusion protein according to any of the preceding items, wherein the fusion protein comprises or consists of an amino acid sequence having the amino acid sequence according to SEQ ID NO: 5, or a variant thereof having at least 80% sequence identity to the amino acid sequence according to SEQ ID NO: 5.
37) The fusion protein according to any of the preceding items, wherein the fusion protein comprises or consists of the amino acid sequence of SEQ ID NO: 5.
38) The fusion protein according to any of the preceding items, wherein the fusion protein inhibits the alternative pathway by preventing binding of factor B or fragments thereof to C3b and recruits factor I, such as wherein the fusion protein recruits the amino acid sequence according to SEQ ID NO: 22.
39) The fusion protein according to any of the preceding items, wherein the fusion protein hinders formation of the alternative pathway C3 convertase, C3bBb and recruits factor I, such as wherein the fusion protein recruits the amino acid sequence according to SEQ ID NO: 22.
40) The fusion protein according to any of the preceding items, wherein the fusion protein inhibits the alternative pathway by preventing binding of factor B or fragments thereof to C3b and activates degradation of C3b by recruiting factor I.
41) The fusion protein according to any of the preceding items, wherein the fusion protein inhibits the alternative pathway by preventing binding of factor B or fragments thereof to C3b and is a co-factor for factor I, such as the co-factor according to the amino acid sequence according to SEQ ID NO: 22.
42) The fusion protein according to any of the preceding items, wherein the fusion protein hinders formation of the alternative pathway C3 convertase, C3bBb and activates degradation of C3b by recruiting factor I, such as recruiting the amino acid sequence according to SEQ ID NO: 22.
43) The fusion protein according to any of the preceding items, wherein the fusion protein hinders formation of the alternative pathway C3 convertase, C3bBb and is a co-factor for factor I, such as the co-factor according to the amino acid sequence according to SEQ ID NO: 22.
44) A polypeptide having a length of less than 220 amino acids, wherein the polypeptide comprises or consists of the amino acid sequence according to SEQ ID NO: 10 or a variant thereof having at least 80% sequence identity to the amino acid sequence according to SEQ ID NO: 10.
45) A polypeptide having a length of less than 350 amino acids, wherein the polypeptide comprises or consists of the amino acid sequence according to SEQ ID NO: 9 or a variant thereof having at least 80% sequence identity to the amino acid sequence according to SEQ ID NO: 9.
46) The polypeptide according to any of items 44 or 45, wherein the polypeptide recruits factor I, such as recruiting the amino acid sequence according to SEQ ID NO: 22.
47) The polypeptide according to any of items 44 or 45, wherein the polypeptide activates degradation of C3b by recruiting factor I, such as by recruiting the amino acid sequence according to SEQ ID NO: 22.
48) The polypeptide according to any of items 44 or 45, wherein the polypeptide is a co-factor for factor I, such as co-factor according to the amino acid sequence according to SEQ ID NO: 22.
49) A nucleic acid encoding the fusion protein of any of items 1-43 or the polypeptides of any of items 44-48.
50) A vector comprising the nucleic acid of item 49.
51) A method of preparing the vector according to item 50, the method comprising:
   a) introducing the vector according to item 50 into a cell;
   b) culturing the cell;
   c) collecting and purifying the vector from the cell; and
   d) optionally, lysing the cell to release the vector from the cell.
52) A host cell comprising the nucleic acid of item 49 or the vector of item 50.
53) A method of preparing the fusion protein of any of items 1-43 or the polypeptide of any of items 44-48, the method comprising:
   a) introducing the nucleic acid according to item 49 or the vector according to item 50 into a host cell;
   b) culturing the cell;
   c) obtaining and optionally purifying the fusion protein or polypeptide from the supernatant; and
   d) optionally, lysing the cell prior to purifying the fusion protein or the polypeptide.
54) A pharmaceutical composition comprising the fusion protein of any of items 1-43 or the polypeptide of any of items 44-48, optionally comprising a pharmaceutically acceptable carrier.
55) A method of treating a disorder associated with complement activation, the method comprising administering a therapeutically effective amount of the fusion protein of any of items 1-43, the polypeptide of any of items 44-48 or the pharmaceutical composition of item 54 to a subject in need thereof.
56) The fusion protein of any of items 1-43, the polypeptides of any of items 44-48 or the pharmaceutical composition according to item 54 for use as a medicament.
57) The fusion protein of any of items 1-43, the polypeptide of any of items 44-48 or the pharmaceutical composition according to item 54 for use in the treatment of a disorder associated with complement activation.
58) The method according to item 55 or the fusion protein, the polypeptide or the pharmaceutical composition for use of item 57, wherein the disorder is selected from the group consisting of ocular diseases, neurological diseases, autoimmune and inflammatory disorders, cancers and infectious diseases.
59) A method of modulating the activity of the complement system, the method comprising: administering to an individual in need thereof a therapeutically effective amount of the fusion protein as defined in any of items 1-43, the polypeptide as defined in any of items 44-48 or the pharmaceutical composition as defined in item 54, thereby modulating the activity of the complement system in the individual in need thereof.

### Examples

### Experimental details

Native C3 was purified from plasma as previously described (Jensen et al., 2018). C3b was generated from purified C3 as described (Pedersen et al., 2020). The glutamatetryptophan-glutamate (EWE) motif was inserted into the hC3Nb1 expression vector and the resulting EWE-hC3Nb1 nanobody was purified as previously described (Jensen et al., 2018). In brief, single domain antibodies were expressed in *E. coli.* Overnight culture was pelleted and cells were resuspended in 20 mM Tris pH 8.5, 500 mM NaCl, 0.5 mM EDTA, 20 mM Imidazole, sonicated and applied to a HisTrap Crude FF (GE Healthcare) column. The protein was eluted by resuspension buffer supplemented with 400 mM Imidazole. The protein was dialyzed overnight against 20 mM acetic acid pH 5.5, 50 mM NaCl, then applied to a Source 15S (GE Healthcare) column and eluted by a linear gradient from 50-350 mM NaCl over 35 column volumes. The protein was finally polished on a Superdex 75 (GE Healthcare) column equilibrated in 20 mM HEPES pH 7.5, 150 mM NaCl.

Mini-FH was purified as previously described (Pedersen et al., 2019). EWEµH and EWEnH were purified using a similar protocol. In brief, the fusion proteins were transiently expressed in HEK293 cells using PEI transfection. The enriched supernatant was harvested, the pH was adjusted to 8.5, and the protein was applied to a HisTrap Excel (GE Healthcare) column. The protein was eluted by addition of 20 mM Tris pH 8.5, 500 mM NaCl, 400 mM Imidazole. The eluted protein was dialyzed against 20 mM Tris pH 8.5, 50 mM NaCl overnight, then applied to a 9 mL Source 15Q (GE Healthcare) column. The protein was eluted by a 60 mL linear gradient from 20-500 mM NaCl. The protein was subsequently applied to a Superdex 200 increase (GE Healthcare) column equilibrated in 20 mM HEPES pH 7.5, 150 mM NaCl.

**Size exclusion chromatography assay.** 200 µg C3b was mixed with a 2-fold molar excess of EWE-hC3Nb1 or left untreated. The mix was diluted to 400 µL in 20 mM HEPES pH 7.5, 150 mM NaCl and applied to a Superdex 200 increase (GE Healthcare) column equilibrated in dilution buffer. SEC assaying binding of EWE-hC3Nb1 to native C3 was performed similarly. Peak fraction were analyzed by SDS PAGE.

**Factor H mediated cleavage of C3b by factor I.** C3b was incubated with a 1.2-fold molar excess of EWE-hC3Nb1 or hC3Nb1 for 5 min. Next, 1% factor I (Complement Technologies) and 0.2% factor H (Complement Technologies) relative to C3b was added. The mix was incubated for the indicated time at 37°C. Reaction buffer was 20 mM HEPES pH 7.5, 150 mM NaCl.

**Comparison of EWEµH with mini-FH.** C3b was mixed with 0.5% factor I (Complement Technologies) and a two-fold molar excess of mini-FH, EWEµH or both EWE-hC3Nb1 and mini-FH. The mix was incubated for the indicated time in 20 mM HEPES pH 7.5, 150 mM NaCl at 37°C.

**Comparison of EWEµH and EWEnH.** C3b was incubated in the indicated molar ratio with EWEµH or EWEnH for 5 min at 4°C, then 1% factor I (Complement Technologies) was added. The reaction buffer was 20 mM HEPES pH 7.5, 150 mM NaCl and reaction temperature was 37°C. The C3b:fusion protein ratios tested were 1:0.5 and 1:2 and were performed in n=2 and n=3 replicates, respectively. Intensity of the bands arising from the C3b a'-chain were quantified using the ImageJ (version 1.52a) software.

**Hemolysis assay.** The indicated inhibitor at 1260, 420, 140, 46.7, 15.6, or 5.2 nM in AP buffer (20 mM HEPES pH 7.4, 150 mM NaCl, 5 mM MgCl₂, 10 mM EGTA, 0.1% gelatin) was mixed with 11% NHS. 20 µL of the inhibitor-NHS mix was incubated with 10 µL 6% Rabbit erythrocytes in AP buffer. The mix was incubated for 2 h at 37°C, then 60 µL cold 0.9% NaCl, 5 mM EDTA was added and the erythrocytes were pelleted by centrifugation at 200 xg for 20 min. 70 µL supernatant was transferred to a 96-well plate and the absorbance at 405 nm was measured using a VICTOR3 Multi-label Plate counter (PerkinElmer)

**Concentration test.** EWEµH or EWEnH was concentrated at 4°C in a vivaspin 500 centrifugal concentrator until clear precipitate was observed in EWEµH concentrator. The concentration of the protein was measured using a Nanodrop ND-1000 spectrophotometer (Seveen Werner). The protein was diluted to 400 µL in 20 mM HEPES pH 7.5, 150 mM NaCl and applied to a Superdex 200 (GE Healthcare) column equilibrated in dilution buffer.

**Bio-layer interferometry experiments.** All experiments were performed in 20 mM HEPES pH 7.5, 150 mm NaCl. All experiments were performed on an Octet RED96 (Fortébio Pall Corporation) instrument operating at 30°C, shaking at 1000 rpm. EWE-hC3Nb1, EWEµH or EWEnH was immobilized on anti-penta his biosensor (Fortébio Pall Corporation) and the sensors were transferred into dilution series of C3b or iC3b. Measurements of 0 nM analyte were subtracted from sensorgrams for normalization and the sensorgrams were fitted to a 1:1 binding model using the GraphPad Prism (version 6.01) software.

### Example 1 - The EWE-hC3Nb1 factor H fusion proteins

EWE-hC3Nb1 was tested for its ability to bind C3 and C3b (Fig 3). EWE-hC3Nb1 conferred a clear shift in elution volume, when mixed with C3b (Fig 3A), but not when mixed with native C3 (Fig 3B), showing that EWE-hC3Nb1 is specific against C3b. A subsequent SDS PAGE of peak fraction confirmed the formation of the EWE-hC3Nb1:C3b complex (Fig 3C).

Design of the EWEµH and EWEnH fusion protein (Fig 4A). The crystal structure of hC3Nb1 in complex with C3b (Jensen et al., 2018) was docked onto the crystal structure of C3b in complex with mini-FH (Xue et al., 2017). In the left panel, CCP1 of mini-FH is not shown. In the right panel, CCP1 and CCP19-20 of mini-FH are not shown. The dashed line indicates a flexible linker joining the C-terminus of the EWE-hC3Nb1 moiety to the N-terminus of CCP2 of the factor H moiety. By comparing the crystal structures of hC3Nb1 or mini-FH bound to C3b, the inventors of the present disclosure realized that the binding sites of hC3Nb1 and CCP1 on C3b overlap extensively (Fig 4B-C). Having realized this overlap in binding site, the inventors hypothesized that a recombinant linkage of EWE-hC3Nb1 to the CCP2-4 and CCP19-20 domains of factor H, might result in an AP inhibitor that mediates cleavage of C3b by factor I.

### Example 2 - Validation of C3b cleavage

Firstly, EWE-hC3Nb1 and hC3Nb1 were tested for their ability to inhibit the factor H mediated cleavage of C3b by factor I. The results shows that both EWE-hC3Nb1 (Fig 5A) and hC3Nb1 (Fig 5B) delays the cleavage of C3b by factor I. Secondly, EWEµH was tested for its ability to sustain cleavage of C3b by factor I. The effect of EWEµH was compared to mini-FH (CCP1-4, 19-20) as well as a mix of both EWE-hC3Nb1 and mini-FH (CCP1-4, 19-20) (Fig 5C). This assay shows that whereas EWEµH sustains the cleavage of C3b, the mix of EWE-hC3Nb1 and mini-FH does not, as such EWEµH mediates cleavage of C3b by factor I.

Further validation was done to compare the EWEnH and EWEµH fusion proteins against each other. Figure 6 shows a comparison of EWEnH and EWEµH. Both fusion proteins mediates cleavage of C3b by factor I in molar ratios of C3b:fusion protein of 1:0.5 (Fig 6A) and 1:2 (Fig 6B). Figure 6C shows a quantification of the remaining C3b at each time point in panels A-B, which indicates that EWEnH is more efficient in sustaining C3b cleavage at both tested molar ratios.

### Example 3 - Comparison of binding affinities and functional properties of the single domain antibody moieties of the fusion proteins

To investigate if the binding affinities of EWE-hC3Nb1 towards C3b was retained when presented in a fusion protein format, **Bio-layer** interferometry-based analysis was performed. Figure 7 shows investigation of the affinity of EWE-hC3Nb1 toward C3b (Fig 7A) and iC3b (Fig 7B), showing that EWE-hC3Nb1 exhibits similar affinity toward the two. Figure 8 shows a bio-layer interferometry based assessment of the affinity of EWEµH toward C3b (Fig 8A) and iC3b (Fig 8B). These data indicate that EWEµH binds C3b with a higher affinity than it binds iC3b. Figure 9 shows the assessment of the interaction of EWEnH with C3b (Fig 9A) and iC3b (Fig 9B). These data shows that EWEnH binds C3b and iC3b with similar affinity. The table in Figure 10 summarizes binding kinetics described in figures 7-9. The fusion EWEµH binds app. 10x stronger to C3b than the single domain antibody EWE-hC3Nb1, whereas it binds with similar affinity towards iC3b. The fusion protein EWEnH shows a slightly lower binding affinity towards C3b and iC3b than the single domain antibody EWE-hC3Nb1.

Having confirmed that the binding affinities of the single domain antibodies were retained, a functional study was conducted to confirm the ability to inhibit AP mediated hemolysis of rabbit erythrocytes. Figure 11 shows that EWEµH, EWEnH, EWE-hC3Nb1, and hC3Nb1 inhibits the AP mediated C3 fragment deposition in 11% NHS. The effect was compared to the broad inhibitor hC3Nb2 (Pedersen et al., 2020), and the inactive hC3Nb1 mutant (W102A) (Jensen et al., 2018).
Overall these data shows that the normal function of the single domain antibodies as AP inhibitors are retained when presented in a fusion protein format.

### Example 4 - Concentration of EWEµH and EWEnH

To investigate the tendency of the two fusion proteins to precipitate at high concentrations, EWEµH and EWEnH were concentrated. The EWEµH started to precipitate at 3.75 mg/mL. EWEnH did not show any precipitation at 22 mg/mL. The concentrated proteins were subjected to SEC, where EWEnH eluted as a monodisperse peak (Fig 12A-B). Showing that the fusion protein EWEnH tolerates ultrafiltration to a higher concentration than EWEµH.

### Sequence overview

**SEQ ID NO: 1**
   EWE-hC3Nb1 
MEWEQVQLVETGGGLVQAGGSLRLSCAASGSIFSINAMGWFRQAPGKEREFVATIN
RSGGRTYYADSVKGRFTISRDNGKNMVYLQMHSLKPEDTAIYYCAAGTGWSPQTDN
EYNYWGQGTQVTVSS
**SEQ ID NO: 2**
   CDR1: SGSIFSINAMG
**SEQ ID NO: 3**
   CDR2: TINRSGGRTY
**SEQ ID NO: 4**
   CDR3: GTGWSPQTDNEYNY
**SEQ ID NO: 5**
   EWEµH 
EWEQVQLVETGGGLVQAGGSLRLSCAASGSIFSINAMGWFRQAPGKEREFVATINRS
GGRTYYADSVKGRFTISRDNGKNMVYLQMHSLKPEDTAIYYCAAGTGWSPQTDNEY
NYWGQGTQVTVSSGGGGSGGGGSGGGGSGGGGSRPCGHPGDTPFGTFTLTGGN
VFEYGVKAVYTCNEGYQLLGEINYRECDTDGWTNDIPICEVVKCLPVTAPENGKIVSS
AMEPDREYHFGQAVRFVCNSGYKIEGDEEMHCSDDGFWSKEKPKCVEISCKSPDVI
NGSPISQKIIYKENERFQYKCNMGYEYSERGDAVCTESGWRPLPSCEEAGGGGGG
GGGGGGKCGPPPPIDNGDITSFPLSVYAPASSVEYQCQNLYQLEGNKRITCRNGQW
SEPPKCLHPCVISREIMENYNIALRWTAKQKLYSRTGESVEFVCKRGYRLSSRSHTLR
TTCWDGKLEYPTCAKRENLYFQG
**SEQ ID NO: 6**
   Polypeptide linker connecting EWE-hC3Nb1 and the CCP2 domain of the factor H moiety:
   GGGGSGGGGSGGGGSGGGGS
**SEQ ID NO: 7**
   EWEnH 
EWEQVQLVETGGGLVQAGGSLRLSCAASGSIFSINAMGWFRQAPGKEREFVATINRS
GGRTYYADSVKGRFTISRDNGKNMVYLQMHSLKPEDTAIYYCAAGTGWSPQTDNEY
NYWGQGTQVTVSSGGGGSGGGGSGGGGSGGGGSRPCGHPGDTPFGTFTLTGGN
VFEYGVKAVYTCNEGYQLLGEINYRECDTDGWTNDIPICEVVKCLPVTAPENGKIVSS
AMEPDREYHFGQAVRFVCNSGYKIEGDEEMHCSDDGFWSKEKPKCVEISCKSPDVI
NGSPISQKIIYKENERFQYKCNMGYEYSERGDAVCTESGWRPLPSCEEA
**SEQ ID NO: 8**
   Mini-FH 
EDCNELPPRRNTEILTGSWSDQTYPEGTQAIYKCRPGYRSLGNIIMVCRKGEWVALN
PLRKCQKRPCGHPGDTPFGTFTLTGGNVFEYGVKAVYTCNEGYQLLGEINYRECDTD
GWTNDIPICEVVKCLPVTAPENGKIVSSAMEPDREYHFGQAVRFVCNSGYKIEGDEE
MHCSDDGFWSKEKPKCVEISCKSPDVINGSPISQKIIYKENERFQYKCNMGYEYSERG
DAVCTESGWRPLPSCEEAGGGGGGGGGGGGKCGPPPPIDNGDITSFPLSVYAPA
SSVEYQCQNLYQLEGNKRITCRNGQWSEPPKCLHPCVISREIMENYNIALRWTAKQK
LYSRTGESVEFVCKRGYRLSSRSHTLRTTCWDGKLEYPTCAK
**SEQ ID NO: 9**
   µH comprising CCP2-CCP4 and CCP19-CCP20 
RPCGHPGDTPFGTFTLTGGNVFEYGVKAVYTCNEGYQLLGEINYRECDTDGWTNDIP
ICEVVKCLPVTAPENGKIVSSAMEPDREYHFGQAVRFVCNSGYKIEGDEEMHCSDDG
FWSKEKPKCVEISCKSPDVINGSPISQKIIYKENERFQYKCNMGYEYSERGDAVCTES
GWRPLPSCEEAGGGGGGGGGGGGKCGPPPPIDNGDITSFPLSVYAPASSVEYQC
QNLYQLEGNKRITCRNGQWSEPPKCLHPCVISREIMENYNIALRWTAKQKLYSRTGE
SVEFVCKRGYRLSSRSHTLRTTCWDGKLEYPTCAK
**SEQ ID NO: 10**
   nH comprising CCP2-CCP4 
RPCGHPGDTPFGTFTLTGGNVFEYGVKAVYTCNEGYQLLGEINYRECDTDGWTNDIP
ICEVVKCLPVTAPENGKIVSSAMEPDREYHFGQAVRFVCNSGYKIEGDEEMHCSDDG
FWSKEKPKCVEISCKSPDVINGSPISQKIIYKENERFQYKCNMGYEYSERGDAVCTES
GWRPLPSCEEA
**SEQ ID NO: 11**
   Factor H CCP2 UniProtKB - P08603 (CFAH_HUMAN) 83-143 
RPCGHPGDTPFGTFTLTGGNVFEYGVKAVYTCNEGYQLLGEINYRECDTDGWTNDIP
ICEV
**SEQ ID NO: 12**
   Factor H CCP3 UniProtKB - P08603 (CFAH_HUMAN) 144-207 
VKCLPVTAPENGKIVSSAMEPDREYHFGQAVRFVCNSGYKIEGDEEMHCSDDGFWS
KEKPKCVE
**SEQ ID NO: 13**
   Factor H CCP4 UniProtKB - P08603 (CFAH_HUMAN) 208-264
   ISCKSPDVINGSPISQKIIYKENERFQYKCNMGYEYSERGDAVCTESGWRPLPSCEE
**SEQ ID NO: 14**
   Factor H CCP19 UniProtKB - P08603 (CFAH_HUMAN) 1107-1165 
GKCGPPPPIDNGDITSFPLSVYAPASSVEYQCQNLYQLEGNKRITCRNGQWSEPPKC
LH
**SEQ ID NO: 15**
   Linker between factor H CCP19 and CCP20 UniProtKB - P08603 (CFAH_HUMAN) 1166-1169
   PCVI
**SEQ ID NO: 16**
   Factor H CCP20 UniProtKB - P08603 (CFAH_HUMAN) 1170-1230 
SREIMENYNIALRWTAKQKLYSRTGESVEFVCKRGYRLSSRSHTLRTTCWDGKLEYP
TCAK
**SEQ ID NO: 17**
   Linker connecting CCP4-CCP19
   AGGGGGGGGGGGG
**SEQ ID NO: 18**
   Factor H CCP19-CCP20 UniProtKB - P08603 (CFAH_HUMAN) 1107-1230 
KCGPPPPIDNGDITSFPLSVYAPASSVEYQCQNLYQLEGNKRITCRNGQWSEPPKCL
HPCVISREIMENYNIALRWTAKQKLYSRTGESVEFVCKRGYRLSSRSHTLRTTCWDG
KLEYPTCAK
**SEQ ID NO: 19**
   C3b alpha chain sequence from rcsb.com [pdb id: 5O35] 749-1662 
SNLDEDIIAEENIVSRSEFPESWLWNVEDLKEPPKNGISTKLMNIFLKDSITTWEILAVS
MSDKKGICVADPFEVTVMQDFFIDLRLPYSVVRNEQVEIRAVLYNYRQNQELKVRVEL
LHNPAFCSLATTKRRHQQTVTIPPKSSLSVPYVIVPLKTGLQEVEVKAAVYHHFISDGV
RKSLKVVPEGIRMNKTVAVRTLDPERLGREGVQKEDIPPADLSDQVPDTESETRILLQ
GTPVAQMTEDAVDAERLKHLIVTPSGCGEQNMIGMTPTVIAVHYLDETEQWEKFGLE
KRQGALELIKKGYTQQLAFRQPSSAFAAFVKRAPSTWLTAYVVKVFSLAVNLIAIDSQV
LCGAVKWLILEKQKPDGVFQEDAPVIHQEMIGGLRNNNEKDMALTAFVLISLQEAKDIC
EEQVNSLPGSITKAGDFLEANYMNLQRSYTVAIAGYALAQMGRLKGPLLNKFLTTAKD
KNRWEDPGKQLYNVEATSYALLALLQLKDFDFVPPVVRWLNEQRYYGGGYGSTQAT
FMVFQALAQYQKDAPDHQELNLDVSLQLPSRSSKITHRIHWESASLLRSEETKENEGF
TVTAEGKGQGTLSVVTMYHAKAKDQLTCNKFDLKVTIKPAPETEKRPQDAKNTMILEI
CTRYRGDQDATMSILDISMMTGFAPDTDDLKQLANGVDRYISKYELDKAFSDRNTLIIY
LDKVSHSEDDCLAFKVHQYFNVELIQPGAVKVYAYYNLEESCTRFYHPEKEDGKLNKL
CRDELCRCAEENCFIQKSDDKVTLEERLDKACEPGVDYVYKTRLVKVQLSNDFDEYIM
 
AIEQTIKSGSDEVQVGQQRTFISPIKCREALKLEEKKHYLMWGLSSDFWGEKPNLSYII
GKDTWVEHWPEEDECQDEENQKQCQDLGAFTESMVVFGCPN
**SEQ ID NO: 20**
   MG7 domain of C3b UniProtKB - P01024 (CO3_HUMAN) 829-933 
FFIDLRLPYSVVRNEQVEIRAVLYNYRQNQELKVRVELLHNPAFCSLATTKRRHQQTV
TIPPKSSLSVPYVIVPLKTGLQEVEVKAAVYHHFISDGVRKSLKVVP
**SEQ ID NO: 21**
   Factor H CCP1 UniProtKB - P08603 (CFAH_HUMAN) 19-82 
EDCNELPPRRNTEILTGSWSDQTYPEGTQAIYKCRPGYRSLGNIIMVCRKGEWVALN
PLRKCQK
**SEQ ID NO: 22**
   Factor I UniProtKB - P05156 (CFAI_HUMAN) 19-583 
KVTYTSQEDLVEKKCLAKKYTHLSCDKVFCQPWQRCIEGTCVCKLPYQCPKNGTAVC
ATNRRSFPTYCQQKSLECLHPGTKFLNNGTCTAEGKFSVSLKHGNTDSEGIVEVKLV
DQDKTMFICKSSWSMREANVACLDLGFQQGADTQRRFKLSDLSINSTECLHVHCRGL
ETSLAECTFTKRRTMGYQDFADVVCYTQKADSPMDDFFQCVNGKYISQMKACDGIN
DCGDQSDELCCKACQGKGFHCKSGVCIPSQYQCNGEVDCITGEDEVGCAGFASVTQ
EETEILTADMDAERRRIKSLLPKLSCGVKNRMHIRRKRIVGGKRAQLGDLPWQVAIKD
ASGITCGGIYIGGCWILTAAHCLRASKTHRYQIWTTVVDWIHPDLKRIVIEYVDRIIFHEN
YNAGTYQNDIALIEMKKDGNKKDCELPRSIPACVPWSPYLFQPNDTCIVSGWGREKD
NERVFSLQWGEVKLISNCSKFYGNRFYEKEMECAGTYDGSIDACKGDSGGPLVCMD
ANNVTYVWGVVSWGENCGKPEFPGVYTKVANYFDWISYHVGRPFISQYNV
**SEQ ID NO 23:**
   hC3Nb1 
MQVQLVETGGGLVQAGGSLRLSCAASGSIFSINAMGWFRQAPGKEREFVATINRSG
GRTYYADSVKGRFTISRDNGKNMVYLQMHSLKPEDTAIYYCAAGTGWSPQTDNEYN
YWGQGTQVTVSS
**SEQ ID NO: 24**
   C3b sequence from rcsb.com [pdb id: 5O35] 23-667

SPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGYLFIQTDKTIYT
PGSTVLYRIFTVNHKLLPVGRTVMVNIENPEGIPVKQDSLSSQNQLGVLPLSWDIPELV
NMGQWKIRAYYENSPQQVFSTEFEVKEYVLPSFEVIVEPTEKFYYIYNEKGLEVTITAR
FLYGKKVEGTAFVIFGIQDGEQRISLPESLKRIPIEDGSGEVVLSRKVLLDGVQNPRAE
DLVGKSLYVSATVILHSGSDMVQAERSGIPIVTSPYQIHFTKTPKYFKPGMPFDLMVFV

TNPDGSPAYRVPVAVQGEDTVQSLTQGDGVAKLSINTHPSQKPLSITVRTKKQELSEA
EQATRTMQALPYSTVGNSNNYLHLSVLRTELRPGETLNVNFLLRMDRAHEAKIRYYTY
LIMNKGRLLKAGRQVREPGQDLVVLPLSITTDFIPSFRLVAYYTLIGASGQREVVADSV
WVDVKDSCVGSLVVKSGQSEDRQPVPGQQMTLKIEGDHGARVVLVAVDKGVFVLNK
KNKLTQSKIWDVVEKADIGCTPGSGKDYAGVFSDAGLTFTSSSGQQTAQRAELQCPQ
PAA

### References

ENGSTROM, G., HEDBLAD, B., BERGLUND, G., JANZON, L. & LINDGARDE, F. 2007. Plasma levels of complement C3 is associated with development of hypertension: a longitudinal cohort study. J Hum Hypertens, 21, 276-82.
JENSEN, R. K., PIHL, R., GADEBERG, T. A. F., JENSEN, J. K., ANDERSEN, K. R., THIEL, S., LAURSEN, N. S. & ANDERSEN, G. R. 2018. A potent complement factor C3-specific nanobody inhibiting multiple functions in the alternative pathway of human and murine complement. J Biol Chem, 293, 6269-6281.
MERLE, N. S., CHURCH, S. E., FREMEAUX-BACCHI, V. & ROUMENINA, L. T. 2015. Complement System Part I - Molecular Mechanisms of Activation and Regulation. Front Immunol, 6, 262.
PEDERSEN, D. V., GADEBERG, T. A. F., THOMAS, C., WANG, Y., JORAM, N., JENSEN, R. K., MAZARAKIS, S. M. M., REVEL, M., EL SISSY, C., PETERSEN, S. V., LINDORFF-LARSEN, K., THIEL, S., LAURSEN, N. S., FREMEAUX-BACCHI, V. & ANDERSEN, G. R. 2019. Structural Basis for Properdin Oligomerization and Convertase Stimulation in the Human Complement System. Front Immunol, 10, 2007.
PEDERSEN, H., JENSEN, R. K., HANSEN, A. G., GADEBERG, T. A. F., THIEL, S., LAURSEN, N. S. & ANDERSEN, G. R. 2020. A C3-specific nanobody that blocks all three activation pathways in the human and murine complement system. J Biol Chem, 295, 8746-8758.
XUE, X., WU, J., RICKLIN, D., FORNERIS, F., DI CRESCENZIO, P., SCHMIDT, C. Q., GRANNEMAN, J., SHARP, T. H., LAMBRIS, J. D. & GROS, P. 2017. Regulator-dependent mechanisms of C3b processing by factor I allow differentiation of immune responses. Nat Struct Mol Biol, 24, 643-651.
Hocking HG, Herbert AP, Kavanagh D, Soares DC, Ferreira VP, Pangburn MK, Uhrín D, Barlow PN. Structure of the N-terminal region of complement factor H and conformational implications of disease-linked sequence variations. J Biol Chem. 2008 Apr 4;283(14):9475-87.

## Claims

1. A fusion protein comprising:
a) a single domain antibody that binds to C3b;
b) a polypeptide comprising or consisting of the amino acid sequence according to SEQ ID NO: 10, or a variant thereof having at least 80% sequence identity to the amino acid sequence according to SEQ ID NO: 10; and
c) a first linker.

2. The fusion protein according to claim 1, wherein the single domain antibody binds an epitope comprised in the MG7 domain of C3b and the N-terminus of the C3b alpha chain, such as an epitope comprised in the amino acid sequence according to SEQ ID NO: 20 and the N-terminus of SEQ ID NO: 19 and/or wherein the single domain antibody competes for binding with the CCP1 domain of factor H, such as wherein the single domain antibody competes for binding with the amino acid sequence according to SEQ ID NO: 21.

3. The fusion protein according to any of the preceding claims, wherein the single domain antibody comprises a CDR1 having the amino acid sequence of SEQ ID NO: 2, a CDR2 having the amino acid sequence of SEQ ID NO: 3 and a CDR3 having the amino acid sequence of SEQ ID NO: 4.

4. The fusion protein according to any of the preceding claims, wherein the single domain antibody comprises a N-terminal motif, such as a bulky N-terminal motif, such as wherein the bulky N-terminal motif creates specificity towards C3b by sterically hindering binding to C3, for example wherein the bulky N-terminal motif is a 3 amino acid sequence comprising or consisting of EWE.

5. The fusion protein according to any of the preceding claims, wherein the first linker is a glycine-serine (GS) linker, such as a GS linker comprising or consisting of an amino acid sequence according to SEQ ID NO: 6.

6. The fusion protein according to any of the preceding claims, wherein the fusion protein inhibits the alternative pathway by preventing binding of factor B or fragments thereof to C3b and recruits factor I and/or wherein the fusion protein hinders formation of the alternative pathway C3 convertase, C3bBb and recruits factor I, such as wherein the fusion protein recruits the amino acid sequence according to SEQ ID NO: 22.

7. The fusion protein according to any of the preceding claims, wherein the fusion protein inhibits the alternative pathway by preventing binding of factor B or fragments thereof to C3b and activates degradation of C3b by recruiting factor I.

8. A polypeptide having a length of less than 220 amino acids, wherein the polypeptide comprises or consists of the amino acid sequence according to SEQ ID NO: 10 or a variant thereof having at least 80% sequence identity to the amino acid sequence according to SEQ ID NO: 10.

9. A polypeptide having a length of less than 350 amino acids, wherein the polypeptide comprises or consists of the amino acid sequence according to SEQ ID NO: 9 or a variant thereof having at least 80% sequence identity to the amino acid sequence according to SEQ ID NO: 9.

10. A nucleic acid encoding the fusion protein of any of claims 1-5 or the polypeptide of any of claims 8-9.

11. A vector comprising the nucleic acid of claim 10.

12. A host cell comprising the nucleic acid of claim 10 or the vector of claim 11.

13. A pharmaceutical composition comprising the fusion protein of any of claims 1-7 or the polypeptide of any of claims 8-9, optionally comprising a pharmaceutically acceptable carrier.

14. The fusion protein of any of claims 1-7, the polypeptide of any of claims 8-9 or the pharmaceutical composition according to claim 13 for use as a medicament.

15. The fusion protein of any of claims 1-7, the polypeptide of any of claims 8-9 or the pharmaceutical composition according to claim 13 for use in the treatment of a disorder associated with complement activation, such as wherein the disorder is selected from the group consisting of ocular diseases, neurological diseases, autoimmune and inflammatory disorders, cancers and infectious diseases.
